(19)

**Europäisches Patentamt**
**European Patent Office**
**Office européen des brevets**

(11) **EP 4 671 273 A1**

(12) **EUROPEAN PATENT APPLICATION**

(43) Date of publication:
**31.12.2025 Bulletin 2026/01**

(21) Application number: **24208869.8**

(22) Date of filing: **25.10.2024**

(51) International Patent Classification (IPC):
**C07K 16/24** (2006.01)    **C07K 16/28** (2006.01)
**C07K 19/00** (2006.01)

(52) Cooperative Patent Classification (CPC):
**C07K 16/2818; C07K 14/54; C07K 16/244;**
A61K 2039/505; A61K 2039/507; C07K 2317/31;
C07K 2317/33; C07K 2317/524; C07K 2317/526;
C07K 2317/55; C07K 2317/565; C07K 2317/567;
C07K 2317/622; C07K 2317/71; C07K 2317/76;
(Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **26.06.2024 EP 24184833**
**27.06.2024 EP 24185173**

(71) Applicant: **Anaveon AG**
**4057 Basel (CH)**

(72) Inventors:
• **HUBER, Christoph**
**4103 Bottmingen (CH)**

• **MURER, Patrizia**
**4058 Basel (CH)**
• **RAU, Alexander**
**4052 Basel (CH)**
• **STOCKER, Christian**
**8005 Zürich (CH)**

(74) Representative: **Schulz Junghans**
**Patentanwälte PartGmbB**
**Großbeerenstraße 71**
**10963 Berlin (DE)**

Remarks:
The complete document including Reference
Table(s) and the Sequence Listing(s) can be
downloaded from the EPO website

(54) **PROXIMITY ACTIVATED INTERLEUKIN 21**

(57)     The invention relates to proximity activated cytokine compounds comprising a PD-1 targeting moiety, and an IL-21 polypeptide covalently linked to an anti-IL-21 antibody-binding domain, which together provide targeted cytokine signaling to PD-1 expressing cells. The invention further relates to nucleic acid and expression vectors encoding proximity activated cytokines, as well as use of such compounds for treating cancer.

EP 4 671 273 A1

(52) Cooperative Patent Classification (CPC): (Cont.)
C07K 2317/92; C07K 2318/10; C07K 2319/00

**Description**

<u>Field</u>

The present invention relates to proximity activated IL-21 compound targeted to PD-1 expressing cells, useful for treating cancer.

<u>Background</u>

IL-21 has shown promise in maintaining T cell function and preventing their dysfunction in chronic antigen exposure conditions, such as cancer. However, the therapeutic benefit of systemic IL-21 administration is limited by its short half-life and pleiotropic effects, including immunosuppressive signaling on antigen-presenting dendritic cells. Targeting IL-21 to PD-1 expressing cells is a promising approach to re-invigorate tumor antigen-specific T cells that have been chronically exposed to tumor antigens. In this context, attenuated cytokines have been designed to maintain their therapeutic benefits while minimizing activation of non-target cells and potential adverse effects.

Various approaches have described the use of attenuated cytokines through mutations designed to decrease their affinity to the cognate cytokine receptor (Shen (2020)). While these approaches have shown potential, they also present a challenge in that attenuation reduces the potency on target cells. This reduction in potency can be problematic if the target cells, such as PD-1 expressing cells, exhibit low levels of the targeting protein. Consequently, there is a pressing need for novel options that maintain therapeutic efficacy of recombinant IL-21 while ensuring adequate potency on target cells.

Based on the above-mentioned state of the art, the objective of the present invention is to provide safe and effective recombinant immunomodulatory cytokine compounds. This objective is attained by the subject-matter of the independent claims of the present specification, with further advantageous embodiments described in the dependent claims, examples, figures and general description of this specification.

<u>Summary of the Invention</u>

The invention relates to proximity activated cytokines (PAC), comprising a moiety targeting a molecule expressed on the surface of a target cell, and a moiety comprising a cytokine covalently linked to an anti-cytokine antibody antigen-binding domain. Such PACs provide enhanced cytokine signaling in the context of proximity to the targeted immune cells. The examples provided herein describe PAC molecules which effectively deliver IL-21 to PD-1-expressing T cells.

A first aspect of the invention relates to PAC comprising an anti-PD-1 antibody antigen-binding domain and a human IL-21 polypeptide linked covalently to an anti-IL-21 antibody antigen-binding domain. In particular embodiments, the inventors provide anti-IL-21 antibodies having a heavy chain variable domain (IL-21-VH) at least 95% similar to SEQ ID NO 003, and a light chain variable domain (IL-21-VL) at least 95% similar to SEQ ID NO 004 which effectively attenuate IL-21 signaling until brought in contact with a target cell by means of the anti-PD-1 specific antibody feature.

Optionally, anti-PD-1 targeting arms that are compatible with the most commonly prescribed immune checkpoint inhibiting agents provide the possibility of combination therapy approaches, allowing improved dosing flexibility. In some embodiments, the PAC comprises a non-blocking PD-1 antibody variable domain described herein, and a cytokine polypeptide joined to an antibody binding domain specific for said cytokine in order to deliver a molecule with attenuated cytokine signaling in the absence of the PD-1 targeting moiety (i.e. a reduced EC50 of cytokine signal on cells lacking PD-1, or with blocked PD-1). In some embodiments, the PAC comprises an antibody antigen-binding domain having a VH, and a VL at least 95% identical to the sequence SEQ ID NO 009, and 010, respectively. In other embodiments, the PAC comprises an antibody antigen-binding domain having a VH, and a VL at least 95% identical to the sequence SEQ ID NO 011, and 012, respectively.

Further encompassed by the invention are PAC comprising an IL-21 linked to an anti-IL-21 antibody binding domain, in a bispecific or scFv format, comprising an alternative targeting moiety antibody domain, such as the variable domain of an antagonist anti-PD-1 antibody, or an antibody targeting a different cell surface receptor on target cell. The invention provides PAC with various linkage formats, and valency, according to those set out in the Examples.

Further aspects of the invention relate to isolated nucleic acid sequences, expression vectors or cells expressing or encoding anti-PD-1, and/or anti-IL21 antibody variable domains and/or PACs as described herein.

The invention further relates to pharmaceutical compositions comprising PAC according to the invention, and their use in treating cancer, in addition to methods of treating cancer patients by administering an effective dose of a PAC according to the invention.

<u>*Terms and definitions*</u>

*General*

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of' or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *IL-21 polypeptide* in the context of the present specification relates to the mature, canonical human IL-21 cytokine polypeptide (Uniprot Q9HBE4 residues 30-162). *IL-21 polypeptides* have pleiotropic roles stimulating cells such as T cells, B cells, and natural killer (NK) cells.

The terms *human IL-21 receptor, IL-21R* in the context of the present specification related to the human IL-21 receptor made of the *IL-21Rα* alpha chain (Uniprot Q9HBE5) in a heterodimer with the common gamma chain. The receptor leads to activation of multiple downstream signaling molecule including STAT3, and results in proliferation and activation of T cells, B cells and NK cells.

The term *having substantially the same biological activity as IL-21* in the context of the present invention relates to the main function of an IL-21 cytokine, i.e. initiating signaling downstream of the human IL-21 receptor (IL-21R). The biological function of an *IL-21 polypeptide* according to the invention is at least 80%, particularly at least 90%, more particularly at least 95% of the percentage of phosphorylated STAT3 (pSTAT3) compared to recombinant human IL-21 (rhIL-21) according to the method of measuring the percent of Jurkat T cells having phosphorylated STAT3 (pSTAT3+), which can be presented as an EC50 value. The biological activity of IL-21 is measured without association with an anti-IL-21 antibody antigen-binding domain in the context of a PAC. biological activity of an IL-21 polypeptide, that may later be covalently associated with antibody to form a PAC.

The term *PD-1* in the context of the present specification relates to the human PD-1 protein, encoded by the *PDCD1* gene, also sometimes referred to as CD279 (Uniprot Q15116).

The term *PD-L1* in the context of the present specification relates to the human PD-L1 protein, encoded by the gene *CD274,* also sometimes referred to as CD274 (Uniprot Q9NZQ7).

The term *proximity activated cytokine (PAC)* in the context of the present specification refers to an immunoconjugate, a recombinant polypeptide molecule comprising at least one cell-surface molecule-targeted antibody binding domain, such as anti-PD-1 antibody antigen binding domains disclosed herein, associated with an anti-cytokine antibody antigen binding domain, and wherein said an anti-cytokine antibody antigen binding domain is itself covalently linked to its target cytokine and attenuates binding of the cytokine to its cognate receptor. In certain embodiments, an anti-PD-1 antibody antigen-binding domain of the *PAC* acts as a targeting domain, and binding of said cytokine to the cell initiates a downstream function in a target immune cell expressing PD-1. In certain embodiments, the anti-cytokine antibody antigen binding domain binds specifically to, and attenuates the signaling of, an IL-21 polypeptide. Examples of targets to which the cell-surface molecule-targeted antibody antigen binding domain may bind on the cell surface include, for example, cell surface cluster of differentiation markers expressed by specific cell subsets, e.g. CD20 on B cells, integrins, antigen receptors, and/or co-stimulatory molecules.

The term *cytokine* refers to a mature interleukin protein, or a variant thereof, capable of binding to a cytokine receptor expressed on the cell surface of an immune cell, and initiating a downstream effect, such as IL-4, or IL-21. A particular embodiment of a cytokine presented in the Examples is the IL-21 polypeptide (SEQ ID NO 015). The term further encompasses variant cytokine polypeptides with improved stability or signaling qualities, examples include, but are not limited to, muteins of IL-21 comprising at least one mutation, or chimeric IL-21 variants comprising deletions, insertions, or chimeric cytokines combining polypeptides derived from two cytokines, such as IL-21 IL-4 chimeric proteins (Shen (2020); Bondensgaard (2007)).

*Sequences*

Sequences similar or homologous (e.g., at least about 70% sequence identity) to the sequences disclosed herein are also part of the invention. In some embodiments, the sequence identity at the amino acid level can be about 80%, 85%, 90%, 91%, 92%, 93%, 94%, 95%, 96%, 97%, 98%, 99% or higher. At the nucleic acid level, the sequence identity can be about 70%, 75%, 80%, 85%, 90%, 91%, 92%, 93%, 94%, 95%, 96%, 97%, 98%, 99% or higher. Alternatively, substantial identity exists when the nucleic acid segments will hybridize under selective hybridization conditions (e.g., very high stringency hybridization conditions), to the complement of the strand. The nucleic acids may be present in whole cells, in a cell lysate, or in a partially purified or substantially pure form.

In the context of the present specification, the terms *sequence identity and percentage of sequence identity* refer to a single quantitative parameter representing the result of a sequence comparison determined by comparing two aligned sequences position by position. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA. Software for performing BLAST analyses is publicly available, e.g., through the National Center for Biotechnology-Information (http:// blast.ncbi.nlm.nih.gov/).

One example for comparison of amino acid sequences is the BLASTP algorithm that uses the default settings: Expect threshold: 10; Word size: 3; Max matches in a query range: 0; Matrix: BLOSUM62; Gap Costs: Existence 11, Extension 1; Compositional adjustments: Conditional compositional score matrix adjustment. One such example for comparison of nucleic acid sequences is the BLASTN algorithm that uses the default settings: Expect threshold: 10; Word size: 28; Max matches in a query range: 0; Match/Mismatch Scores: 1.-2; Gap costs: Linear. Unless stated otherwise, sequence identity values provided herein refer to the value obtained using the BLAST suite of programs (Altschul et al., J. Mol. Biol. 215:403-410 (1990)) using the above identified default parameters for protein and nucleic acid comparison, respectively.

Reference to identical sequences without specification of a percentage value implies 100% identical sequences (i.e. the same sequence).

Particular embodiments make use of the sequences as disclosed herein (i.e. 100% identical).

*General Biochemistry: Peptides, Amino Acid Sequences*

The term *polypeptide* in the context of the present specification relates to a molecule consisting of 50 or more amino acids that form a linear chain wherein the amino acids are connected by peptide bonds. The amino acid sequence of a polypeptide may represent the amino acid sequence of a whole (as found physiologically) protein or fragments thereof. The term "polypeptides" and "protein" are used interchangeably herein and include proteins and fragments thereof. Polypeptides are disclosed herein as amino acid residue sequences.

The term *peptide* or oligopeptide in the context of the present specification relates to a molecule consisting of up to 50 amino acids, in particular 8 to 30 amino acids, more particularly 8 to 15 amino acids, that form a linear chain wherein the amino acids are connected by peptide bonds.

Amino acid residue sequences are given from amino to carboxyl terminus. Capital letters for sequence positions refer to L-amino acids in the one-letter code (Stryer, Biochemistry, 3rd ed. p. 21). Lower case letters for amino acid sequence positions refer to the corresponding D- or (2R)-amino acids. Sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

The term *variant* refers to a polypeptide that differs from a reference polypeptide, but retains essential properties. A typical variant of a polypeptide differs in its primary amino acid sequence from another, reference polypeptide. Generally, differences are limited so that the sequences of the reference polypeptide and the variant are closely similar overall and, in many regions, identical. A variant and reference polypeptide may differ in amino acid sequence by one or more modifications (e.g., substitutions, additions, and/or deletions). A substituted or inserted amino acid residue may or may not be one encoded by the genetic code. A variant of a polypeptide may be naturally occurring such as an allelic variant, or it may be a variant that is not known to occur naturally.

In the context of the present specification, the term dimer refers to a unit consisting of two subunits.

In the context of the present specification, the term *homodimer* refers to a dimer comprised of two subunits that are either identical or are highly similar members of the same class of subunits.

In the context of the present specification, the term *amino acid linker* or *peptide linker* refers to a polypeptide of variable length that is used to connect two polypeptides in order to generate a single chain polypeptide. Exemplary embodiments of linkers useful for practicing the invention specified herein are oligopeptide chains consisting of 1, 2, 3, 4, 5, 10, 20, 30, 40 or 50 amino acids.

There is no constraint on the amino acid composition of the linker. In certain embodiments, the linker consists of amino acids selected from the group of S, P, N, A, H, G, Q, T, E, and K. In certain embodiments, the linker consists of amino acids selected from the group of G S, A and D. An important characteristic of the conjugate peptide linkers as specified above are low immunogenicity, and a peptide length that allows the domains which are joined by the linker, to interact to form a functional entity as disclosed herein. In particular desirable embodiments of the domain peptide linkers specified above, the sequences are primarily made up of stretches of amino acids such as glycine (G) and serine (S). A non-limiting example of an amino acid linker is a monomer or di-, tri-or tetramer of a peptide motif composed of three or four glycine and one serine.

Any embodiments relating peptide linkers as disclosed herein, encompass structures in which amino acids with similar characteristics are exchanged, for example, the amino acids V, L, I, P, S, C, or M may replace G, S, or S, and D may be replaced by E.

*General Molecular Biology: Nucleic Acid Sequences, Expression*

The term gene refers to a polynucleotide containing at least one open reading frame (ORF) that is capable of encoding a particular polypeptide or protein after being transcribed and translated. A polynucleotide sequence can be used to identify larger fragments or full-length coding sequences of the gene with which they are associated. Methods of isolating larger fragment sequences are known to those of skill in the art.

The term *transgene* in the context of the present specification relates to a gene or genetic material that has been transferred from one organism to another. In the present context, the term may also refer to transfer of the natural or physiologically intact variant of a genetic sequence into tissue of a patient where it is missing. It may further refer to transfer of a natural encoded sequence the expression of which is driven by a promoter absent or silenced in the targeted tissue.

The term *recombinant* in the context of the present specification relates to a nucleic acid, which is the product of one or several steps of cloning, restriction and/or ligation and which is different from the naturally occurring nucleic acid. A recombinant virus particle comprises a recombinant nucleic acid.

The terms *gene expression* or *expression*, or alternatively the term *gene product,* may refer to either of, or both of, the processes - and products thereof - of generation of nucleic acids (RNA) or the generation of a peptide or polypeptide, also referred to transcription and translation, respectively, or any of the intermediate processes that regulate the processing of genetic information to yield polypeptide products. The term *gene expression* may also be applied to the transcription and processing of a RNA gene product, for example a regulatory RNA or a structural (e.g. ribosomal) RNA. If an expressed polynucleotide is derived from genomic DNA, expression may include splicing of the mRNA in a eukaryotic cell. Expression may be assayed both on the level of transcription and translation, in other words mRNA and/or protein product.

The term *Nucleotides* in the context of the present specification relates to nucleic acid or nucleic acid analogue building blocks, oligomers of which are capable of forming selective hybrids with RNA or DNA oligomers on the basis of base pairing. The term *nucleotides* in this context includes the classic ribonucleotide building blocks adenosine, guanosine, uridine (and ribosylthymine), cytidine, the classic deoxyribonucleotides deoxyadenosine, deoxyguanosine, thymidine, deoxyuridine and deoxycytidine. It further includes analogues of nucleic acids such as phosphothioates, 2'O-methylphosphothioates, *peptide nucleic acids* (PNA; N-(2-aminoethyl)-glycine units linked by peptide linkage, with the nucleobase attached to the alpha-carbon of the glycine) or *locked nucleic acids* (LNA; 2'O, 4'C methylene bridged RNA building blocks). Wherever reference is made herein to a *hybridizing sequence*, such hybridizing sequence may be composed of any of the above nucleotides, or mixtures thereof.

The term *nucleic acid expression vector* in the context of the present specification relates to a plasmid, a viral genome or an RNA, which is used to transfect (in case of a plasmid or an RNA) or transduce (in case of a viral genome) a target cell with a certain gene of interest, or -in the case of an RNA construct being transfected- to translate the corresponding protein of interest from a transfected mRNA. For vectors operating on the level of transcription and subsequent translation, the gene of interest is under control of a promoter sequence and the promoter sequence is operational inside the target cell, thus, the gene of interest is transcribed either constitutively or in response to a stimulus or dependent on the cell's status. In certain embodiments, the viral genome is packaged into a capsid to become a viral vector, which is able to transduce the target cell.

The term *mRNA* in the context of the present specification relates to a polynucleotide consisting of ribonucleotides that can be processed by eukaryote ribosomes. The mRNA comprises a coding sequence that can be translated into the encoded (poly)peptide sequence, here an antibody domain, or recombinant cytokine antibody polypeptide as described elsewhere herein. The mRNA also comprises an untranslated region (5'UTR) that aids in its binding to and processing by the ribosome ("Shine Dalgarno region). mRNA may comprise a 5' cap (7-methylguanosine) and a 3' polyadenosine (poly(A)) tail region.

*Binding; Binders, Ligands, Antibodies:*

If not specified more narrowly in the Detailed Description of the Invention, reference to binders and ligands encompasses antibodies, antibody-like molecules and aptamers as defined in the following paragraphs.

The term *specific binding* in the context of the present invention refers to a property of ligands that bind to their target with a certain affinity and target specificity. The affinity of such a ligand is indicated by the dissociation constant of the ligand. A specifically reactive ligand has a dissociation constant of $\leq 10^{-8}$ mol/L (particularly $\leq 10^{-9}$ mol/L) when binding to its target, but a dissociation constant at least three orders of magnitude higher in its interaction with a molecule having a globally similar chemical composition as the target, but a different three-dimensional structure.

In the context of the present specification, the term *dissociation constant ($K_D$)* is used in its meaning known in the art of chemistry and physics; it refers to an equilibrium constant that measures the propensy of a complex composed of [in most cases, two] different components to dissociate reversibly into its constituent components. The complex can be e.g. an antibody-antigen complex AbAg composed of antibody Ab and antigen Ag. $K_D$ is expressed in molar concentration [mol/l] and corresponds to the concentration of [Ab] at which half of the binding sites of [Ag] are occupied, in other words, the concentration of unbound [Ab] equals the concentration of the [AbAg] complex. The dissociation constant can be calculated according to the following formula:

$$K_D = \frac{[Ab] * [Ag]}{[AbAg]}$$

[Ab]: concentration of antibody; [Ag]: concentration of antigen; [AbAg]: concentration of antibody-antigen complex

In the context of the present specification, the terms *off-rate* ($K_{off}$;[1/sec]) and *on-rate* ($K_{on}$; [L/(sec*mol)]) are used in their meaning known in the art of chemistry and physics; they refer to a rate constant that measures the dissociation ($K_{off}$) or association ($K_{on}$) of an antibody with its target antigen. $K_{off}$ and $K_{on}$ can be experimentally determined using methods well established in the art. A method for determining the $K_{off}$ and $K_{on}$ of an antibody employs surface plasmon resonance. This is the principle behind biosensor systems such as the Biacore® or the ProteOn® system. They can also be used to determine the dissociation constant $K_D$ by using the following formula:

$$K_D = \frac{[K_{off}]}{[K_{on}]}$$

The natural upper limit for the on-rate $K_{on}$ is $10^9$ L/(sec*mol).

In the context of the present specification, the term *antibody* refers to whole antibodies including but not limited to immunoglobulin type G (IgG), type A (IgA), type D (IgD), type E (IgE) or type M (IgM), any antigen-binding fragment, or single chains thereof, and related or derived constructs. A whole antibody is a glycoprotein comprising at least two heavy (H) chains and two light (L) chains inter-connected by disulphide bonds. Each heavy chain is comprised of a heavy chain variable region (VH) and a heavy chain constant region (CH). The heavy chain constant region of IgG is comprised of three domains, $C_H1$, $C_H2$ and $C_H3$. Each light chain is comprised of a light chain variable region (abbreviated herein as VL) and a light chain constant region (CL). The light chain constant region is comprised of one domain, CL. The variable regions of the heavy and light chains associate to create an antibody binding domain that interacts with an antigen. The binding specificity of the antibody is largely determined by the complementarily determining regions (CDR). The CDR as sometimes referred by their position on the light chain (LCDR, CDR-L), or the heavy chain (HCDR, CDR-H), and by their number 1, 2, or 3 designating their position in the variable domain region. If not otherwise indicated, numbering systems identifying amino acid residues in the CDR regions as used in this specification use the standard international ImMunoGeneTics information system, which the skilled person will recognize are slightly different from those assigned under alternative equivalent numbering systems such as Chothia, or Kabat. Mutations in the Fc domain that confer Fc receptor silencing, or enhanced chain pairing, are names by their commonly used Kabat numbering labels. The constant regions of the antibodies may mediate the binding of the immunoglobulin to host tissues or factors, including various cells of the immune system (e.g., effector cells) and the first component of the classical complement system. The term antibody encompasses a so-called nanobody or single domain antibody, an antibody fragment consisting of a single monomeric variable antibody domain.

The term *antibody antigen-binding domain* in the context of the specification refers to a combination of a heavy chain variable domain sequence, and a light chain variable domain polypeptide sequence, which when associated provide a functional antibody *antigen-binding domain* specific for the non-blocking PD-1 epitopes of the invention. This may be in the context of a full antibody heavy chain polypeptide associated with a light chain polypeptide, or alternatively, an scFv.

In the context of the present specification, the term *fragment crystallizable (Fc) region* is used in its meaning known in the art of cell biology and immunology; it refers to a fraction of an antibody comprising, if applied to IgG, two identical heavy chain fragments consisting of a $C_H2$ and a $C_H3$ domain, covalently linked by disulphide bonds. The term *Fab fragment (Fab)* refers to the region of antibody that binds to antigen, comprising the constant and variable domains of a heavy and light chain pair.

In the context of the present specification, the term *single-chain variable fragment (scFv)* relates to a fusion protein of the variable regions of the heavy (VH) and light chains (VL) of an immunoglobulin, resulting in an antibody-like, high affinity to the target from a single polypeptide chain. The VH and VL chains of the scFv are connected through a short linker peptide of ten to about 25 amino acids [Huston et al. (1988). PNAS 85 (16): 5879-5883] The linker can either connect the N-terminus of the VH with the C-terminus of the VL (VL-VH), or adopt the reverse configuration (VH-VL). In the context of the specification, the term *single-chain variable fragment (scFv)* refers to a fusion protein in which the variable regions of the light chain, and heavy chain variable domains of the Fab fragment of a monoclonal antibody are joined by means of a flexible peptide linker. Multimeric *scFv* can be created by joining two or more pairs of heavy and light chain domains by peptide linkers.

*(Cancer) Immunotherapy*

In the context of the present specification, the term *anti-PD-1 antagonist antibody* is meant to encompass an agent, particularly an antibody (or antibody-like molecule) capable of disrupting the signal cascade leading to T cell inhibition after T cell activation as part of what is known in the art the immune checkpoint mechanism. Non-limiting examples include antibodies to PD-1 (Uniprot Q15116), exemplified by the clinically available antibody drugs nivolumab (Bristol-Myers Squibb; CAS No 946414-94-4), pembrolizumab (Merck Inc.; CAS No. 1374853-91-4), dostarlimab (Tesaro; CAS No 2022215-59-2), sintilimab (Eli Lilly, InnoVent Biologics; CAS No 2072873-06-2), tislelizumab (BeiGene; CAS No 1858168-59-8), cemiplimab (CAS No 1801342-60-8), cetrelimab (CAS No 2050478-92-5), sasanlimab (CAS No 2206792-50-7), toripalimab (CAS No, 1924598-82-2), zeluvalimab (CAS No 2315361-37-4) or ezabenlimab (CAS No 2249882-54-8). *Anti-PD-1 antagonist antibodies* inhibit binding of PD-1 to the receptors PD-L1 and PD-L2. Binding of PD-1 to PD-L1 and PD-L2 inhibits T cell activation, survival and proliferation. An anti-PD-1 antagonist antibody disrupts this signal release the brakes provided by this so-called checkpoint inhibition negative feedback.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

The term *cancer* as used in the context of the present specification relates to malignant neoplastic disease; the terms "cancer" and "malignant neoplastic disease" are used synonymously herein. They specifically include carcinoma (epithelial derived cancer), sarcoma (connective tissue derived cancer), lymphoma and leukemia, germ-cell derived tumors and blastomas. Particular alternatives of any of the aspects and embodiments disclosed herein are directed at the use of the compounds and compositions of the invention in treatment of solid tumors. Other alternatives of any of the aspects and embodiments disclosed herein are directed at the use of the combinations of the invention in treatment of liquid cancers such as myelogenous or granulocytic leukemia, particularly AML, lymphatic, lymphocytic, or lymphoblastic leukemia and lymphoma, polycythemia vera or erythema.

As used herein, the term *treating* or *treatment* of any disease or disorder (e.g. cancer) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

Detailed Description of the Invention

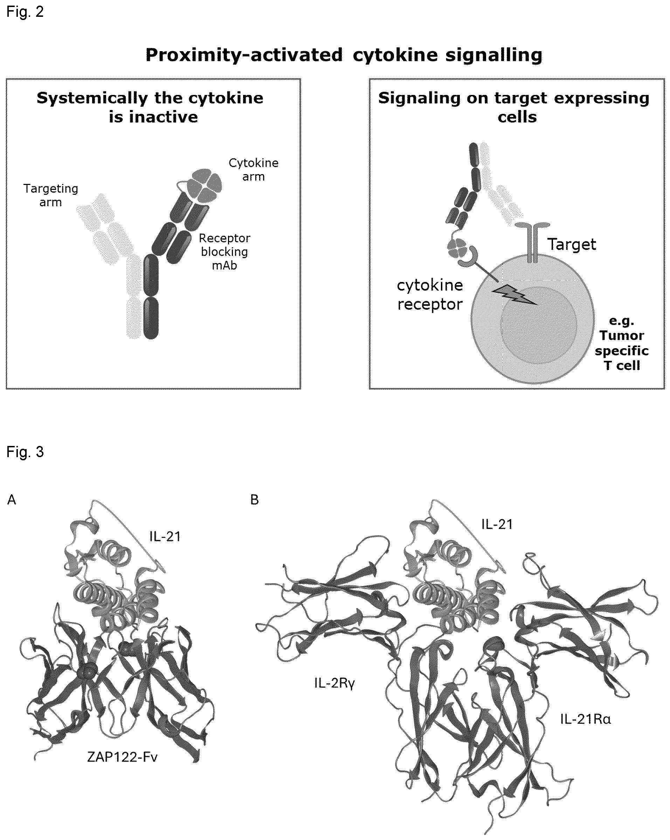

The invention presented herein aims to increase the safety and efficacy of a recombinant cytokine by developing Proximity Activated Cytokine (PAC) compounds. These compounds comprise one arm targeting a cell membrane antigen (e.g., PD-1) and the second arm being an antibody-cytokine fusion protein. The cytokine arm includes an antibody specifically binding to the cytokine at its receptor binding site, optimized to block systemic cytokine signaling while permitting signaling when the bispecific antibody is bound to the cell membrane antigen (Fig. 2).

A first aspect of the invention relates to a PAC comprising an anti-PD1 antibody antigen binding domain capable of binding to PD-1 comprising a heavy chain variable domain (PD1-VH), and a light chain variable domain (PD1-VL), an anti-IL-21 antibody antigen binding domain, and a human IL-21 polypeptide. The IL-21 polypeptide is covalently linked to the either IL-21-VL, or the IL-21-VH, to provide a single contiguous recombinant polypeptide.

The inventors explored a variety of anti-IL-21 antibodies to determine that a heavy chain variable domain (IL-21-VH) comprising a polypeptide at least 95% identical to the sequence SEQ ID NO 003; and a light chain variable domain (IL-21-VL) comprising a polypeptide at least 95% identical to the sequence SEQ ID NO 004 provides effective attenuation of IL-21 signaling in various formats, which is lifted when the PAC is in close proximity to a PD-1+ cell providing more focused effects on target cells. In some embodiments, the PAC comprises an anti-IL-21 antibody antigen binding domain IL-21-VH consisting of polypeptide at least 95% identical to the sequence SEQ ID NO 003, and an IL-21-VL consisting of a polypeptide at least 95% identical to the sequence SEQ ID NO 004.

The inventors found that IL-21 polypeptides may be linked to the anti-IL-21 antibody antigen binding domain in a variety of formats, for example an N-terminus of the IL-21-VL can be covalently linked to a C-terminus of IL-21 polypeptide by a peptide linker, or the N-terminus and the C-terminus of the IL-21 polypeptide can be embedded in a complementarity determining region (CDR) of the heavy or light chain. In the latter case, should the PAC comprise an anti-IL-21 antibody antigen binding domain IL-21-VH having a polypeptide at least 95% identical to the sequence SEQ ID NO 003, or an IL-21-VL consisting of a polypeptide at least 95% identical to the sequence SEQ ID NO 004, this sequence identity relates to the antibody-derived sequences only, and any IL-21 polypeptides and linkers in an embedded construct are not taken into account in terms of sequence identity.

In some embodiments, the PAC comprises an IL-21 polypeptide at least 98% identical to SEQ ID NO 015, and having the same biological activity of inducing STAT3 signaling. In other embodiments, the PAC comprises an IL-21 polypeptide consisting of SEQ ID NO 015.

In some embodiments of the PAC according to the invention, the anti-IL-21 antibody antigen binding domain comprises an IL-21-VH polypeptide having at least one amino acid substitution disclosed in Table 3, or Table 4 compared to the sequence SEQ ID NO 003. In some embodiments of the PAC, the anti-IL-21 antibody antigen binding domain comprises an IL-21-VL polypeptide having at least one amino acid substitution disclosed in Table 3, or Table 4 compared to the sequence SEQ ID NO 004. In some embodiments, the anti-IL-21 antibody antigen binding domain comprises a combination of the substitutions listed in Table 3 compared to SEQ ID NO 003 and SEQ ID NO 004. In some embodiments, the anti-IL-21 antibody antigen binding domain comprises a VH and VL pair listed in Table 3. In some embodiments, the anti-IL-21 antibody antigen binding domain comprises a VH and VL pair listed in Table 4.

The combinations of mutations listed in Tables 3, and/or 4 provide clones with altered affinity for IL-21 compared to the published sequence of ZAP122, which enable compatibility with different peptide linker arrangements joining the IL-21 polypeptide to the anti-IL-21 antibody antigen binding domain, and have favorable manufacturing and developability qualities such as a lack of post-translational modification sites in the CDR. The resulting PAC shield IL-21 from interactions with cells other than PD-1+ target cells expressing the IL-21 receptor

In some embodiments of the PAC according to the invention, the anti-PD-1 antibody antigen binding domain does not significantly impede the simultaneous binding of pembrolizumab, or nivolumab to a PD-1+ cell. This can be confirmed in a competition assay whereby incubating pembrolizumab, or nivolumab together with a cell, does not significantly inhibit subsequent binding of the PAC to said cell (or vice versa). In particular embodiments, a competition assay using preincubation with pembrolizumab, or nivolumab gives no more than 20% inhibition of PAC binding.

In some embodiments of the PAC according to the invention, the PD1-VH comprises a variable domain comprising a polypeptide at least 95% identical to the sequence SEQ ID NO 009, and the PD1-VL comprises a variable domain comprising a polypeptide at least 95% identical to the sequence SEQ ID NO 010. In some embodiments, the PD1-VH comprises a variable domain consisting of a polypeptide at least 95% identical to the sequence SEQ ID NO 009, and the PD1-VL comprises a variable domain consisting of a polypeptide at least 95% identical to the sequence SEQ ID NO 010.

In some embodiments of the PAC according to the invention, the PD1-VH of the PD-1 targeting moiety comprises a variable domain comprising at least one amino acid substitution disclosed in Table 6 compared to the sequence SEQ ID NO 009; and the PD1-VL comprises a variable domain comprising SEQ ID NO 010. In some embodiments of the PAC according to the invention, the PD1-VH of the PD-1 targeting moiety comprises a variable domain comprising 2, 3, 4, or 5 acid substitutions disclosed in Table 6 compared to the sequence SEQ ID NO 009; and the PD1-VL comprises a variable domain comprising SEQ ID NO 010. In some embodiments, the anti-PD-1 antibody antigen binding domain comprises a VH and VL pair listed in Table 6.

In some embodiments of the PAC according to the invention, the PD1-VH comprises a variable domain consisting of SEQ ID NO 009, and the PD1-VL comprises a variable domain consisting of SEQ ID NO 010.

In some embodiments of the PAC according to the invention, the PD1-VH of the PD-1 targeting moiety comprises a variable domain comprising at least one amino acid substitution disclosed in Table 6 compared to the sequence SEQ ID NO 011; and the PD1-VL comprises a variable domain comprising SEQ ID NO 012. In other embodiments, the PD1-VH comprises at least one amino acid substitution disclosed in Table 7 compared to the sequence SEQ ID NO 011; and the PD1-VL comprises at least one amino acid substitution disclosed in Table 7 compared to the sequence SEQ ID NO 012. In some embodiments of the PAC according to the invention, the PD1-VH of the PD-1 targeting moiety comprises a variable domain comprising at least 2, 3, 4, or 5 amino acid substitution disclosed in Table 7 compared to the sequence SEQ ID NO 011; and/or the PD1-VL comprises a variable domain comprising at least 2, 3, 4, or 5 amino acid substitutions disclosed in Table 7 compared to the sequence SEQ ID NO 012. In some embodiments, the anti-PD-1 antibody antigen binding domain comprises a VH and VL pair listed in Table 7.

In some embodiments of the PAC according to the invention, the PD1-VH comprises a variable domain consisting of SEQ ID NO 011, and the PD1-VL comprises a variable domain consisting of SEQ ID NO 012.

In certain embodiments of the PAC according to the invention, the PAC is targeted to PD-1+ cells using an antibody antigen-binding domain of an antagonist anti-PD-1 antibody, such as pembrolizumab or nivolumab. In some embodiments of the PAC according to the invention, the PD1-VH comprises a variable domain comprising or consisting of SEQ ID NO 020, and the PD1-VL comprises a variable domain comprising or consisting of SEQ ID NO 021. In some embodiments of the PAC according to the invention, the PD1-VH comprises a variable domain comprising or consisting of SEQ ID NO 022, and the PD1-VL comprises a variable domain comprising or consisting of SEQ ID NO 023.

In certain embodiments of the PAC according to the invention, the PAC is targeted to PD-1+ cells by means of an antibody antigen-binding domain derived from an antagonist anti-PD-1 antibody having a lambda light chain, ensuring correct HC/LC pairing in a heterotetrametric format with an IL-21-VH having a kappa IL-21VL. In some embodiments, the PD1-VH comprises a variable domain comprising or consisting of SEQ ID NO 035, and the PD1-VL comprises a variable domain comprising or consisting of SEQ ID NO 036. In some embodiments, the PD1-VH comprises a variable domain comprising or consisting of SEQ ID NO 037, and the PD1-VL comprises a variable domain comprising or consisting of SEQ ID NO 038. In some embodiments, the PD1-VH comprises a variable domain comprising or consisting of SEQ ID NO 39 and the PD1-VL comprises a variable domain comprising or consisting of SEQ ID NO 040.

In some embodiments of the PAC according to the invention, an N-terminus of the IL-21-VL is covalently linked to a C-terminus of IL-21 polypeptide by a peptide linker. In particular embodiments the antibody and the IL-21 are joined by a peptide linker which as a length of 2 to 20 amino acids. In some embodiments, the linker is 2-10 amino acids in length. In some embodiments, the linker is ($G_4S$).

In some embodiments of the PAC according to the invention, both the N-terminus and the C-terminus of the IL-21 polypeptide are embedded within the complementarity determining region (CDR) of the IL-21-VL. In some embodiments, the IL-21 polypeptide is inserted between the antibody light chain was cleaved between the CDR-L1 residues Q27 and G28 residues at, or corresponding to the antibody VL SEQ ID NO 004. In some embodiments, the IL-21 is inserted within the variable domain and is sandwiched between a peptide linker of 2 to 25 amino acids at either end. In certain embodiments, the IL-21 polypeptide is embedded within the CDR of the anti-IL-21 antibody antigen binding domain, preceded and followed by a GGSG peptide linker (SEQ ID NO 028).

In other embodiments, embedding of IL-21 is in the CDR-L3 between the residues in the variable light chain domain at the residues, or at the residues corresponding (aligning with) N92 and S93 of SEQ ID NO 004.

In some embodiments of the PAC according to the invention, an N-terminus of the IL-21-VH of the anti-IL-21 antibody antigen binding domain is covalently linked to a C-terminus of IL-21 polypeptide by a peptide linker. In particular embodiments, the peptide linker has a length of 10 to 25 amino acids.

In particular embodiments of the PAC according to the invention, the PAC comprises an IgG Fc. In particular embodiments, the PAC comprises an IgG Fc having knob-in-holes mutations to enhance IgG chain pairing as described in references: Atwell, 1997; Merchant, 1998. In particular embodiments, the PAC comprises two different HCs, the HC of an anti-PD-1 antibody, and the HC of an anti-IL-21 antibody, engineered to form a heterodimer by inserting knobs-into-hole mutations knobs: S354C, T366W; holes: Y349C, T366S, L368A, Y407V (according to the Kabat numbering system).

In particular embodiments, the Fc portion of the bispecific is an IgG1 Fc which contains Fc silencing mutations L234A, L235A, P329A (according to the Kabat numbering system).

In certain embodiments of the PAC according to the invention, the PAC is a heterotetrameric IgG, also sometimes referred to as a bispecific antibody. In some embodiments, the PAC comprises a first antibody heavy and light chain heterodimer comprising the anti-PD-1 antibody antigen-binding domain, and a second antibody heavy and light chain antibody heterodimer comprising the anti-IL-21 antibody antigen-binding domain. The heavy chain, or the light chain of the anti-IL-21 portion are covalently linked to the IL-21 polypeptide. The anti-PD-1 portion and the anti-IL-21 portion of the heterotetrameric IgG may be specifically associated with by means of a Crossmab arrangement, a kappa lambda heterodimer pairing, or other bispecific formats known to the skilled person. Such methods can be found, for example in Klein (2016), Klein (2019), and Bönisch (2017), incorporated in their entirety herein by reference.

*Vectors, cells, expressing domains or immunoconjugates according to the invention*

Another aspect of the invention relates to an isolated nucleic acid encoding the PAC according to any one of the embodiments of the immunoconjugate aspect of the invention. Another aspect of the invention relates to an expression vector comprising said isolated nucleic acid. The invention further encompasses a host cell comprising said nucleic acid or said expression vector. In particular embodiments, the isolated nucleic acid is comprised in an expression vector under control of a promoter operable in a mammalian cell.

Another aspect of the invention relates to a combination medicament comprising

i) A PAC as specified in any one of the aspects or embodiments of the invention herein, and
ii) an anti-PD-1 antagonist antibody.

In particular embodiments the anti-PD1 antagonist antibody is selected from the list consisting of nivolumab, pembrolizumab, dostarlimab, sintilimab, tislelizumab, cemiplimab, cetrelimab, sasanlimab, toripalimab, zeluvalimab and ezabenlimab. In more particular embodiments, the antibody is cemiplimab, dostarlimab, zeluvalimab, tiselizumab, ezabenlimab, toripalimab, cetrelimab, nivolumab or pembrolizumab.

In particular embodiments of the combination medicament, the PAC comprises an anti-PD-1 antibody antigen-binding domain which does not significantly inhibit binding of nivolumab or pembrolizumab binding to PD-1 positive cells, such as those disclosed in tables 6 and 7.

*Medical treatment and medical use*

Similarly, within the scope of the present invention is a method of treating cancer in a patient in need thereof, comprising administering to the patient an effective dose of an immunoconjugate according to the above aspects and embodiments of the description.

In some embodiments, the PAC is a heterotetrameric immunoglobulin characterized by two heavy chains and two light chains, one pair of which forms an IL-21 binding ligand and is fused to an IL-21 polypeptide (as in Fig. 2). In other embodiments, the PAC consists of a bivalent targeting domain antibody binding a cell-surface molecule, particularly an associated pair of heavy and light chain heterodimers of an anti-PD-1 antibody, joined by means of a peptide linker to a scFv comprising anti-IL-21 antibody heavy and light chain variable domains, one of which anti-IL-21 domain is joined (optionally by means of peptide linkers) to an IL-21 polypeptide.

Another aspect of the invention, is a PAC comprising a (nivolumab, pembrolizumab) non-blocking anti-PD-1 binding domain according to the invention, for use in a patient who is receiving concurrent treatment with an anti-PD1 antagonist antibody selected from the list consisting of nivolumab, pembrolizumab, dostarlimab, sintilimab, tislelizumab, cemiplimab, cetrelimab, sasanlimab, toripalimab, zeluvalimab and ezabenlimab. In more particular embodiments, the antibody is cemiplimab, dostarlimab, zeluvalimab, tiselizumab, ezabenlimab, toripalimab, cetrelimab, nivolumab or pembrolizumab.

Another aspect of the invention is an anti-PD1 antagonist antibody selected from the list consisting of nivolumab, pembrolizumab, dostarlimab, sintilimab, tislelizumab, cemiplimab, cetrelimab, sasanlimab, toripalimab, zeluvalimab and ezabenlimab for use in a patient receiving administration of a PAC comprising a non-blocking PD-1 antibody antigen-binding domain according to the invention. This encompasses use in a patient scheduled to soon receive treatment with an immunoconjugate within a medically relevant window, particularly within 6 weeks of the anti-PD-1 administration. In certain embodiments, the anti-PD-1 antagonist antibody is provided for use in a patient who has been administered the immunoconjugate according to the invention with the previous month.

The invention further encompasses nivolumab for use in a patient who is receiving administration of a PAC comprising a non-blocking PD-1 antibody antigen-binding domain according to the invention. The invention further encompasses pembrolizumab for use in a patient who is receiving administration of a PAC according to the invention.

Similarly, the invention encompasses methods of treatment of a patient having been diagnosed with a cancer. This method entails administering to the patient an effective amount of a PAC as identified herein, its pharmaceutically acceptable salt, as specified in detail herein. Optionally in combination with an anti-PD1 antagonist antibody, when a PAC comprises a PD-1 non-blocking targeting arm.

In particular embodiments, the same subject is co-administered, within a medically relevant window, anti-PD1 antagonist antibody is selected from the list consisting of nivolumab, pembrolizumab, dostarlimab, sintilimab, tislelizumab, cemiplimab, cetrelimab, sasanlimab toripalimab, zeluvalimab or ezabenlimab. In more particular embodiments, the antibody is cemiplimab, dostarlimab, zeluvalimab, tiselizumab, ezabenlimab, toripalimab, cetrelimab, nivolumab or pembrolizumab.

*Pharmaceutical Compositions, Administration/Dosage Forms and Salts*

According to one aspect of the compound according to the invention, the PACS according to the invention are provided as a pharmaceutical composition, pharmaceutical administration form, or pharmaceutical dosage form, said pharmaceutical composition, pharmaceutical administration form, or pharmaceutical dosage form comprising the immunoconjugate of the present invention and at least one pharmaceutically acceptable carrier, diluent or excipient.

In certain embodiments of the invention, the PACS of the present invention is typically formulated into pharmaceutical dosage forms to provide an easily controllable dosage of the drug and to give the patient an elegant and easily handled product.

The invention further encompasses a pharmaceutical composition comprising PACS of the present invention, and a pharmaceutically acceptable carrier. In further embodiments, the composition comprises at least two pharmaceutically acceptable carriers, such as those described herein.

Certain embodiments of the invention relate to a dosage form for parenteral administration, such as subcutaneous, intravenous, intrahepatic or intramuscular injection forms. Optionally, a pharmaceutically acceptable carrier and/or excipient may be present.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

*Method of Manufacture and Method of Treatment according to the invention*

The invention further encompasses, as an additional aspect, the use of PACs as specified in detail above, for use in a method of manufacture of a medicament for the treatment or prevention of a cancer.

Wherever alternatives for single separable features such as, for example, an isotype protein or coding sequence, or cancer are laid out herein as "embodiments", it is to be understood that such alternatives may be combined freely to form discrete embodiments of the invention disclosed herein. Thus, any of the alternative embodiments for a PAC may be combined with any of the alternative embodiments of anti-PD-1 antagonist antibody and these combinations may be combined with any medical indication mentioned herein.

The invention further encompasses the following items.

A. An anti-PD-1 antibody antigen-binding domain, comprising a heavy chain variable domain polypeptide (PD1-VH), and a light chain variable domain polypeptide (PD1-VL), characterized in that:

- the PD1-VH comprises, or consists of a variable domain comprising, or consisting of SEQ ID NO 009; and

- the PD1-VL comprises, or consists of a variable domain comprising at least one amino acid substitution disclosed in Table 6 compared to the sequence SEQ ID NO 010.

B. The anti-PD-1 antibody antigen-binding domain according to item A, wherein the PD1-VL comprises, or consists of a variable domain comprising at least 2, 3, 4, or 5 amino acid substitutions disclosed in Table 6 compared to the sequence SEQ ID NO 010.

C. An anti-PD-1 antibody antigen-binding domain comprising a heavy chain variable domain polypeptide (PD1-VH), and a light chain variable domain polypeptide (PD1-VL), characterized in that:

- the PD1-VH comprises, or consists of a variable domain comprising at least one amino acid substitution disclosed in Table 7 compared to the sequence SEQ ID NO 011; and
- the PD1-VL comprises, or consists of a variable domain comprising at least one amino acid substitution disclosed in Table 7 compared to the sequence SEQ ID NO 012.

D. An antibody comprising at least one anti-PD-1 antibody antigen binding domain as specified in any one of the items A to C.

E. A proximity activated cytokine (PAC) comprising:

- the anti-PD-1 antibody antigen-binding domain as specified in item A - C,

- a cytokine, and

- an anti-cytokine antibody antigen-binding domain capable of binding to said human cytokine, wherein the anti-cytokine antibody antigen-binding domain comprises an antibody heavy chain variable domain polypeptide (cytokine-VH), and an antibody light chain variable domain polypeptide (cytokine-VL),

and wherein the cytokine is covalently linked to the cytokine-VL, or the cytokine-VH of the anti-cytokine antibody antigen binding domain to provide a single contiguous recombinant polypeptide.

F. The PAC according to item E, wherein the PAC is a heterotetrametric IgG, comprising

- a first antibody heavy and light chain heterodimer comprising the anti-PD-1 antibody antigen-binding domain as specified in item A, B or C; and

- a second antibody heavy and light chain heterodimer comprising the anti-cytokine antibody antigen-binding domain.

G. An anti-IL-21 antibody antigen-binding domain, comprising a heavy chain variable domain polypeptide (IL-21-VH), and a light chain variable domain polypeptide (IL-21-VL), characterized in that:

- the IL-21-VH comprises, or consists of a variable domain polypeptide comprising at least one amino acid substitution disclosed in Table 3, or 4 compared to the sequence SEQ ID NO 003; and

- the IL-21-VL comprises, or consists of a variable domain polypeptide comprising at least one amino acid substitution disclosed in Table 3, or 4 compared to the sequence SEQ ID NO 004.

H. The anti-IL-21 antibody antigen binding domain according to item G, further comprising an IL-21 polypeptide covalently linked to the IL-21-VL, or the IL-21-VH of the anti-IL-21 antibody antigen-binding domain to provide a single contiguous recombinant polypeptide.

I. The PAC according to item E or F, wherein the anti-cytokine antibody antigen-binding domain comprises, or consists of the anti-IL-21 antibody antigen-binding domains as specified in item G or H.

J. A PAC comprising:

- an antibody antigen-binding domain specific for a molecule expressed on the surface of a cell;

- an IL-21 polypeptide; and

- an anti-IL-21 antibody antigen-binding domain as specified in item G.
  wherein said IL-21 polypeptide is covalently linked to the IL-21-VL, or the IL-21-VH of said anti-IL-21 antibody antigen-binding domain to provide a single contiguous recombinant polypeptide.

The invention is further illustrated by the following examples and figures, from which further embodiments and advantages can be drawn. These examples are meant to illustrate the invention but not to limit its scope.

*Description of the Figures*

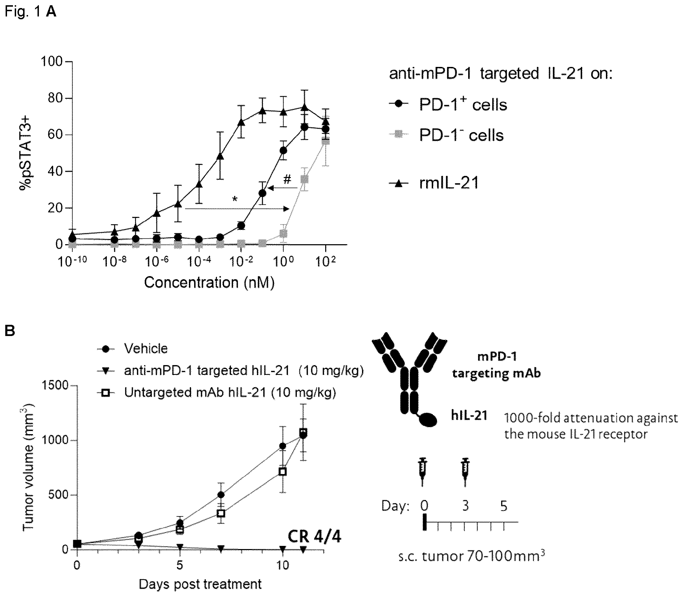

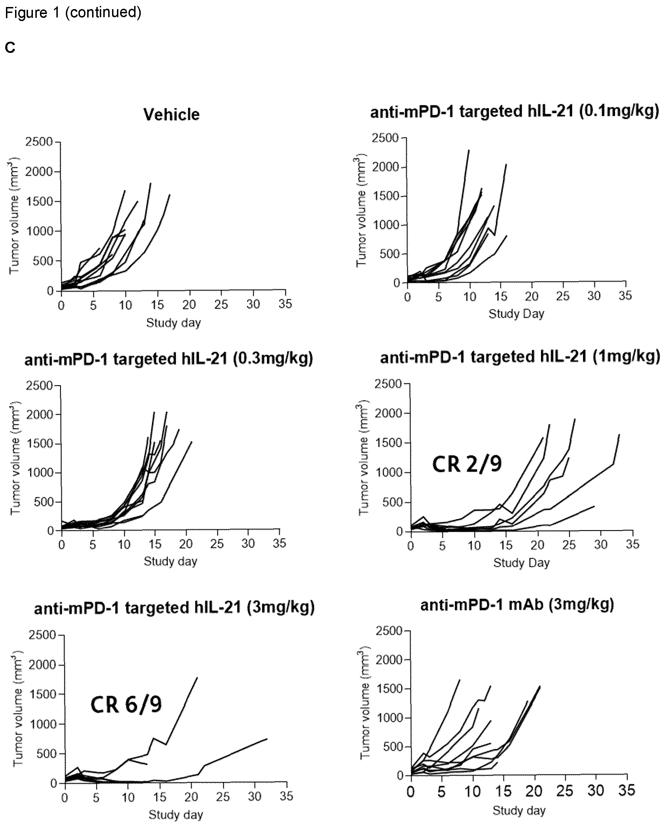

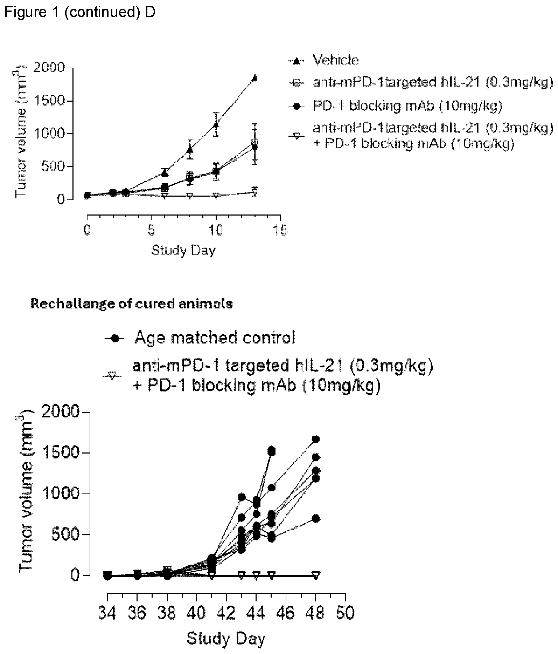

Fig. 1 shows A) STAT3 phosphorylation by the anti-mPD-1 targeted hIL-21 in mouse PD-1+ and PD-1- CD8 T Cells. Recombinant mouse IL-21 (rmIL-21) used as control compound. * Potency attenuation compared to rmIL-21. # Gain of potency on PD-1+ cells. n=3 mean +/- SD. B) Tumor volume over time of C57BL/6 mice bearing s.c. MC38 tumors. Mice were treated i.v. with vehicle, the anti-mPD-1 hIL-21 fusion protein or an untargeted antibody fused to hIL-21 on day 0 (tumor volume average 70-100mm$^3$) and day 3. C) Dose escalation study using the anti-mPD-1 hIL-21 fusion protein as treatment agent in s.c. MC38 tumor bearing mice. Compound or vehicle were injected on days 0 (tumor volume average 70-100mm$^3$) and day 3. The control antibody targeting mPD-1 was used as control treatment. D) Tumor volume over time of C57BL/6 mice bearing s.c. MC38 tumors. Mice were treated i.v. with vehicle, anti-mPD-1 hIL-21 fusion protein (0.3 mg/kg, i.v.), PD-1 blocking mAb (10mg/kg, i.p.) or the combination of the two treatments on day 0 (tumor volume average 70-100mm$^3$) and day 3.

Fig. 2 shows design approach for a proximity activated cytokine.

Fig. 3 shows structure of Interaction between IL-21 (light grey, ribbon) and the ZAP122-Fv (dark grey, ribbon). The N-termini amino acid of ZAP122-Fv light and heavy chain are shown as spheres. B: Modelling of interaction between IL-21 (light grey, ribbon) and the IL-21R$\alpha$ and IL-2R$\gamma$ (PDB: 8ENT, dark grey, ribbon).

Fig. 4 shows PAC compounds with different valency for cell membrane antigen targeting moiety to cytokine arm.

Examples

*Example 1: Targeting IL-21 signaling to PD-1 expressing cells*

The inventors confirmed the principle that attenuated IL-21 can maintain therapeutic benefits while minimizing potential adverse events in a mouse model of cancer using an anti-mouse PD-1 (mPD-1) antibody fused to human IL-21 (hIL-21). The mouse PD-1 antibody used was a non-antagonistic clone lacking checkpoint inhibitor activity. The hIL-21 has an approximately 1000-fold lower affinity for the mouse IL-21 receptor compared to mouse IL-21. The anti-mouse PD-1 fusion protein to hIL-21 induced a stronger IL-21R signaling in PD-1$^+$ CD8 T cells from mouse splenocytes (measured by detecting phosphorylated STAT3 (pSTAT3)) than in PD-1$^-$CD8 cells (Figure 1A). The compound is a model for targeted delivery and localized activation of IL-21. Targeting of hIL-21 to PD-1+ cells lead to potent tumor growth inhibition in mice bearing s.c. MC38 tumors (4/4 complete responses, Fig. 1 B) compared to treatment with vehicle or non-PD-1 targeted antibody-IL-21 fusion protein. The anti-mPD-1 hIL-21 fusion protein showed a dose-dependent antitumoral response in the s.c. MC38 mouse model. The control anti-PD-1 antibody showed only moderate tumor growth inhibition compared to vehicle treatment (Fig. 1C). The combination treatment of a suboptimal dose (0.3mg/kg) of anti-mPD-1 hIL-21 fusion protein with a mouse PD-1 blocking antibody (10mg/kg i.p.) led to a strong synergistic effect of the two agents on MC38 tumor growth inhibition, compared to vehicle and each of the single agents (Fig. 1D). Cured mice that were re-challenged with the MC38 cell line rejected the tumor, indicating that the treatment induced anti-tumor immune memory.

Given the promising preclinical data obtained with the surrogate compound, which demonstrated effective targeted delivery of attenuated IL-21 to PD-1 expressing cells and significant anti-tumor activity in a mouse model, the focus of the present invention shifts to the development of a human therapeutic. The inventors aimed to provide IL-21 signaling specifically and safely to target cells by means of Proximity Activated Cytokine (PAC) compounds. These compounds are bispecific antibodies with one arm targeting a cell membrane antigen (e.g., PD-1) and the second arm being an antibody-cytokine fusion protein. The cytokine arm includes an antibody specifically binding to the cytokine at its receptor binding site, optimized to block systemic cytokine signaling while permitting signaling when the bispecific antibody is bound to the cell membrane antigen (Fig. 2).

*Example 2: Identification of anti-IL-21 binding antibodies blocking IL-21R$\alpha$*

Four anti-human IL-21 (hIL-21) antibodies that were described as IL-21 receptor alpha (IL-21R$\alpha$) blockers were identified (Table 1). In a first experiment, the binding of each antibody to recombinant hIL-21 (rhIL-21) and blockade of the IL-21Ra was assessed by surface plasmon resonance analysis (SPR, Biacore T200, Cytiva). The biotinylated rhIL-21-Fc-Avitag (AcroBiosystems) was captured on a CAP chip (Xantec, RGD200M) at flow rate of 10 $\mu$l/ml to approximately 185 RU. The anti-hIL-21 human IgG1 antibodies were used as the first analyte, diluted in HBSTE running buffer (Xantec) at a flow rate of 30 $\mu$l/min with 180s association and 600s dissociation. After single cycle kinetic at 5 different concentrations (100, 20, 4, 0.8 and 0.16 nM) the recombinant IL-21Ra-Fc (rIL-21Rα, AcroBiosystems) was used as the second analyte diluted at 200nM in HBSTE Buffer. Regeneration was carried out with 50 mM NaOH + 1M NaCl, pH 14 at flow rate 30 μL/min. Single cycle kinetic (SCK) of the rIL-21Rα-Fc (with the same setting as the anti-IL-21 antibodies) FYP014, ZAP122, MIZ926 and LKK631 showed high binding affinity (1:1 fitting) to immobilized rhIL-21. After injection of the second analyte, rIL-21Ra-Fc, no binding was observed after the SCK of ZAP122, MIZ926 or LKK631, indicating that binding of the IL-21Rα to IL-21 was blocked. Association of the rIL-21Ra-Fc was observed after SCK with a control antibody that does not bind to hIL-21.

Table 1 Anti-IL-21 antibodies, VH and VL sequences showed binding to rhIL-21 by SPR. No IL-21Rα binding to the antibody-cytokine complexes with ZAP122, LKK631 and MIZ926 was observed by SPR.

| Antibody name | Source | VH | VL | Binding to immobilized rhIL-21 | Blocked binding of rhIL-21Rα-Fc |
|---|---|---|---|---|---|
| FYP014 | EP3103813A1 (clone 366.328.10.63) | SEQ ID NO 001 | SEQ ID NO 002 | Yes | No |
| ZAP122 | EP3103813A1 (clone 366.552.11.31) | SEQ ID NO 003 | SEQ ID NO 004 | Yes | Yes |
| LKK631 | EP3128997B1 (clone 9H10) | SEQ ID NO 005 | SEQ ID NO 006 | Yes | Yes |
| MIZ926 | EP3128997B1 (clone 8B6) | SEQ ID NO 006 | SEQ ID NO 007 | Yes | Yes |

The functional neutralization efficacy of the anti-hIL21 antibodies of Table 1 was tested by analyzing pSTAT3 phosphorylation on Jurkat cells after stimulation with rhIL-21 alone or in complex with increasing concentrations of anti-hIL21 antibodies. Jurkat cells were pre-stimulated overnight in growth medium containing 0.01 μg/ml phorbol-12-myristate-13-acetate (PMA) and 1.0 μg/ml Ionomycin. A dilution series starting at 10 nM of the anti-hIL-21 antibodies was prepared and incubated with fixed concentration of 0.1 nM rhIL-21 in cell culture medium for 30 minutes. Jurkat cells were washed twice with PBS before being stimulated for 15 minutes with antibody-rhIL-21 complexes. Phosphorylated STAT3 positive (pSTAT3+) cells were detected by flow cytometry. Cells that were incubated with rhIL-21 without blocking antibodies were approximately 82% pSTAT3+ compared to unstimulated cells. The percentage of pSTAT3+ cells was reduced upon pre-complexing of rhIL-21 with the antibodies of Table 1. ZAP122 reduced the pSTAT3+ cells to less than 10% at concentrations higher than 1nM and FYP014 at concentrations higher than 0.16 nM (Table 2). However, the antibody FYP014 did not block hIL-21 interaction with hIL-21Rα and exhibited unspecific binding to the recombinant IL-21Rα (not shown) and was not suitable for the development of a PAC. The antibody ZAP122 showed potent IL-21 signaling neutralization and blockade of the IL-21Rα binding.

Table 2 Percentage pSTAT3+ Jurkat cells after stimulation with rhIL-21 pre-complexed with anti-hIL-21 antibodies

| Antibody concentration (nM) | ZAP122 | MIZ926 | LKK631 | FYP014 |
|---|---|---|---|---|
| 0 | 82 | 82.5 | 82.1 | 82.7 |
| 0.1012 | 33.3 | 75.1 | 81 | 59.4 |
| 0.1602 | 33.2 | 72.4 | 72.4 | 6.07 |
| 0.2536 | 18.2 | 70.7 | 71.4 | 4.93 |
| 0.4015 | 22.8 | 69.9 | 71.5 | 4.39 |
| 0.6355 | 33 | 68.5 | 65.6 | 5.32 |
| 1.006 | 10.8 | 65.3 | 69.5 | 5.9 |
| 1.5925 | 9.01 | 64.8 | 58.7 | 4.78 |
| 2.5209 | 6.78 | 64.5 | 55.1 | 5.16 |
| 3.9906 | 5.32 | 61.8 | 42 | 4.64 |
| 6.3171 | 8.64 | 57.5 | 44.5 | 3.99 |

(continued)

| Antibody concentration (nM) | ZAP122 | MIZ926 | LKK631 | FYP014 |
|---|---|---|---|---|
| 10 | 6.63 | 57.5 | 41.1 | 3.83 |

*Example 3. Crystal structure of ZAP122 Fab in complex with rhIL-21*

The ZAP122-Fab and the human interleukin-21 (IL-21) antigen were mixed at 1:1.15 molar ratio and incubated for 16h at 4 °C. The complex was purified by size exclusion chromatography (Superdex 200 16/60) in PBS. Crystals of the Fab-IL-21 complex were obtained in sitting drop at a protein concentrated of 10.5 mg/ml in 0.2 M Sodium malonate pH 7.0, 20% w/v PEG 3,350. Two IL-21-ZAP122 data sets were collected on the same crystal using a Eiger2 XE 16M detector on beamline i03 at Diamond synchrotron indexed and integrated using their automated pipeline (XIA2_DIALS). The data sets were combined and scaled using AIMLESS (CCP4i2). A previous low resolution (3.3 Å) crystal structure, model was used for the molecular replacement in MOLREP (CCP4i2). A unique solution was found with four copies in the asymmetric unit. The model was given 50 cycles of jelly body refinement with the data using REFMAC5 (CCP4i2). The difference electron density map calculated after initial refinement was examined for movement of any loops or side chains. The loops were fitted into the electron density using COOT and refined using REFMAC5 (CCP4i2). Water molecules were then added using the water placement option in COOT and refined using REFMAC5 (CCP4i2). The structural geometry of the final protein model was checked using MOLPROBITY (Davis et al., 2007) and checked using Maestro. The structure of IL-21 bound to ZAP122 has been solved at 2.26 Å resolution (Fig. 3 A). There are four IL-21-ZAP122 complexes per asymmetric unit. All four complexes have the same IL-21 : ZAP122 (CDR regions) interface. The interaction interface involves helix A (involved in IL-21R$\alpha$ binding) and D (involved in IL-2R$\gamma$ binding) of IL-21 (Fig. 3 B).

*Example 4. Variation of binding affinity of ZAP122 to rhIL-21*

To generate PAC compounds with a broad range of affinity of the anti-IL-21 antibody, an affinity de-maturation of ZAP122 was performed through germline backmutation and alanine substitution in the CDR3 regions. 10 mutants of ZAP122 were generated by mutating amino acids that were different from the closest germline sequences of the light chain (IGKV1-12, IGKV1D-12) and of the heavy chain (IGHV4-4, IGHV4-59) in the framework regions and the CDR1 and CDR2. In addition, alanine substitution of the amino acids of the CDR regions and removal of a post translational modification (PTM) site in the CDR-L3 by N92Q, N92S or N92A generated 19 additional mutants. These 29 ZAP122 variants were expressed as Fab fragments in CHO cells. Purity and protein production yield after protein A purification are reported in Table 3.

The binding kinetics of the ZAP122 mutants to rhIL-21 was assessed by SPR (Biacore 8K, GE Healthcare). Anti-human IgG Fc (Jackson) was immobilized on an amine reactive CM5 chip (GE Healthcare) using 1xHBS-EP+ running buffer (GE Healthcare). Recombinant hIL-21-Fc (AcroBiosystems) were captured at flow rate 10 $\mu$l/min with 30 s contact time. ZAP122 Fab and the ZAP122 mutant Fabs were run as analytes, diluted in 1xHBS-EP+ running buffer (GE Healthcare) with 240 s association and 3600 s dissociation at flow rate 30 $\mu$l/min. Regeneration was carried out with 10 mM Glycine-HCl, pH 1.5. The association constant (ka), dissociation rate constant (kd) and equilibrium dissociation constant (K$_D$) are reported in Table 3.

Table 3. Purity and yield after protein A of ZAP122 clone mutants expressed as Fab fragments. Binding kinetics of each clone measured by SPR. *The $k_d$ value is beyond the limitation of the instrument (1.00E-05), the results here showed < 1.00E-05 for reference only. # KD was estimated using one ka measurement.

| Anti-hIL-21 clone | Mutation | | Purity | Yield | $k_a$ | $k_d$ | $K_D$# |
|---|---|---|---|---|---|---|---|
| | VH | VL | % | mg/L | (1/Ms) | (1/s) | (M) |
| ZAP122 wt | - | - | 97.46 | 93.87 | 3.77E+05 | <1.00E-05* | <2.65E-11 |
| ZAP122m1 | | Q37Y | 93.69 | 45.96 | 3.69E+05 | 1.91E-05 | 5.17E-11 |
| ZAP122m2 | R75K | | 94.52 | 79.83 | 3.63E+05 | 1.23E-05 | 3.40E-11 |
| ZAP122m3 | | V50A | 95.29 | 87.59 | 3.70E+05 | 9.30E-05 | 2.51E-10 |
| ZAP122m4 | | Q89A | 92.69 | 52.19 | 7.24E+04 | 2.53E-04 | 3.49E-09 |
| ZAP122m5 | | Q90A | 97.42 | 5.00 | 1.14E+05 | 1.00E-05 | 8.80E-11 |
| ZAP122m6 | | N92A | 94.67 | 60.53 | 3.79E+05 | 1.43E-05 | 3.79E-11 |

(continued)

| Anti-hIL-21 clone | Mutation | | Purity | Yield | $k_a$ | $k_d$ | $K_D$# |
|---|---|---|---|---|---|---|---|
| | VH | VL | % | mg/L | (1/Ms) | (1/s) | (M) |
| ZAP122m7 | | S93A | 92.99 | 51.6 | 3.87E+05 | <1.00E-05* | <2.58E-11 |
| ZAP122m8 | | F94A | 98.54 | 8.66 | 2.64E+05 | 2.51E-03 | 9.49E-09 |
| ZAP122m9 | | P95A | 98.93 | 3.35 | 2.38E+05 | 5.93E-05 | 2.49E-10 |
| ZAP122m10 | | L96A | 87.72 | 8.71 | 2.56E+05 | 6.13E-04 | 2.40E-09 |
| ZAP122m11 | | T97A | 86.82 | 8.89 | 3.63E+05 | 1.46E-05 | 4.04E-11 |
| ZAP122m12 | D32Y | | 98.99 | 70.6 | 4.81E+05 | 4.24E-03 | 8.83E-09 |
| ZAP122m13 | F33Y | | 97.38 | 103.35 | 3.16E+05 | 1.78E-05 | 5.65E-11 |
| ZAP122m14 | G35S | | 97.27 | 109.48 | 2.60E+05 | 2.63E-04 | 1.01E-09 |
| ZAP122m15 | S52Y | | 98.96 | 13.89 | 5.74E+05 | <1.00E-05* | <1.74E-11 |
| ZAP122m16 | S53Y | | 97.9 | 62.11 | 3.53E+05 | <1.00E-05* | <2.83 E-11 |
| ZAP122m17 | R54S | | 97.79 | 66.52 | 3.34E+05 | 2.81E-05 | 8.42E-11 |
| ZAP122m18 | R65S | | 99.51 | 3.09 | 3.02E+05 | 1.40E-05 | 4.63E-11 |
| ZAP122m19 | R97A | | 98.51 | 26.82 | 1.27E+05 | 7.75E-04 | 6.08E-09 |
| ZAP122m20 | S98A | | 97.08 | 28.23 | 4.46E+05 | 1.94E-05 | 4.35E-11 |
| ZAP122m21 | G100A | | 98.01 | 87.66 | 5.05E+05 | 1.10E-03 | 2.19E-09 |
| ZAP122m22 | V101A | | 98.87 | 80.7 | 3.89E+05 | 3.40E-03 | 8.73E-09 |
| ZAP122m23 | T102A | | 98.49 | 76.6 | 2.40E+05 | 3.29E-05 | 1.37E-10 |
| ZAP122m24 | D103A | | 97.76 | 101.77 | No binding | | |
| ZAP122m25 | F104A | | 89.12 | 7.73 | 1.05E+05 | 5.90E-04 | 5.60E-09 |
| ZAP122m26 | D105A | | 96.71 | 42.36 | 2.33E+05 | 7.01E-04 | 3.01E-09 |
| ZAP122m27 | F106A | | 98.86 | 49.74 | 4.14E+05 | <1.00E-05* | <2.42E-11 |
| ZAP122p1 | | N92Q | 98.09 | 38.92 | 3.57E+05 | 1.38E-05 | 3.86E-11 |
| ZAP122p2 | | N92S | 97.09 | 42.5 | 3.48E+05 | 1.14E-05 | 3.28E-11 |

Combining the mutations of the clones with moderately decreased affinity and good productivity (yield and purity after one step purification) with additional variants to remove potential post-translation modification sites, and/or removal of rare occurring residues (VL-M4L, L4 occurs in 57% of germline (vs. 34%); VL-N92S, VL-N92Q, VL-N92A, removal of N-glycosylation site; VH-F33Y and VH-F106A; removal of hydrophobic patch; VH-R54S, R occurs in approximately 1% (S occurs in 30% of the germline sequences (VH4-59) and did not significantly impair binding of clone m17); VH-R65S, R occurs in less than 1% (S occurs in 27% of the germline sequences (VH4-59) and did not significantly impair binding of clone m18)) resulted in 9 additional clones, expressed as Fab fragments (Table 4). The binding kinetics of the ZAP122 mutants to rhIL-21 was assessed by SPR (Biacore T200, Cytiva). His-capture Kit (28995056, Cytiva) was used to prepare a His-capture CM5 chip (29104988, Cytiva). rhIL-21-His (AB235875-1001, Abcam) was captured at flow rate 10 μl/min until a capture level of 110-120 RU was reached. Serial dilutions of ZAP122 Fab and the ZAP122 mutant Fabs were run as analytes, diluted in 1xHBSTE running buffer (B HBST10, Xantec) as single cycle kinetic with 120 s association and 600 s dissociation at flow rate 30 μl/min. Regeneration was carried out with 10 mM Glycine-HCl, pH 1.5. The association constant (ka), dissociation rate constant (kd) and equilibrium dissociation constant ($K_D$) are reported in Table 3.

Table 4 Association constant (ka), dissociation rate constant (kd) and equilibrium dissociation constant ($K_D$) of mutant variants of ZAP122. Nd: not determinable

| Anti-hIL-21 clone | Mutation compared to SEQ ID NO 003/004 | | $k_a$ | $k_d$ | $K_D$ |
|---|---|---|---|---|---|
| | VH | VL | | | |
| ZAP122 | wt | wt | 2.21E+05 | 6.91E-05 | 3.13E-10 |
| VPH777 | F33Y, R54S, R65S, T102A | M4L, N92S | 1.4E+05 | 1.0E-04 | 7.3E-10 |
| PAI969 | F33Y, R54S, R65S, T102A, F106A | M4L, N92S | 1.4E+05 | 1.4E-04 | 9.8E-10 |
| UER756 | F33Y, R54S, R65S, F106A | M4L, N92S | 1.4E+05 | 1.1E-04 | 8.1E-10 |
| MEK904 | F33Y, S53Y, R65S, T102A | M4L, N92S | 2.1E+05 | 1.5E-04 | 6.9E-10 |
| CLJ397 | F33Y, S53Y, R65S, T102A, F106A | M4L, N92S | 2.7E+05 | 1.5E-04 | 5.5E-10 |
| JSQ278 | F33Y, S53Y, R65S, F106A | M4L, N92S | 2.7E+05 | 1.5E-04 | 5.6E-10 |
| EJT070 | F33Y, S53Y, R54S, R65S, T102A | M4L, N92S | 2.5E+05 | 2.0E-04 | 8.2E-10 |
| OWS965 | F33Y, S53Y, R54S, R65S, T102A, F106A | M4L, N92S | 8.7E+04 | 1.7E-04 | 2.0E-09 |
| RPD212 | F33Y, S53Y, R54S, R65S, F106A | M4L, N92S | 1.3E+05 | 1.5E-04 | 1.1E-09 |
| ZXI489 | F33Y, S53Y, R65S, F106W | M4L, N92S, F94A | 6.88E+05 | 4.84E-03 | 7.04E-09 |
| IFQ920 | T102A | F94A | 2.81E+05 | 2.33E-03 | 8.28E-09 |
| IOO459 | F33Y, T102A | F94A | 1.42E+06 | 2.22E-02 | 1.56E-08 |
| EMV293 | Fully germline back mutation | | nd | nd | nd |

Three additional clones (ZXI489, IFQ920, IOO459) were designed by crystal structure guided mutagenesis and expressed as Fab fragments (Table 4). The binding kinetics of the ZAP122 mutants to rhIL-21 was assessed by SPR (Biacore T200, Cytiva). His capture Kit (28995056, Cytiva) was used to prepare a His-capture CM5 chip (29104988, Cytiva). rhIL-21-His (AB235875-1001, Abcam) was captured at flow rate 10 µl/min until a capture level of 110-120 RU was reached. Serial dilutions of ZAP122 Fab and the ZAP122 mutant Fabs were run as analytes, diluted in 1xHBSTE running buffer (B HBST10, Xantec) as single cycle kinetic with 120 s association and 1800 s dissociation at flow rate 30 µl/min. Regeneration was carried out with 10 mM Glycine-HCl, pH 1.5. The association constant (ka), dissociation rate constant (kd) and equilibrium dissociation constant ($K_D$) are reported in Table 4.

The antibodies described in this example with varying affinity for cytokine IL-21 were utilized to generate PAC compounds. The optimization of the antibody's affinity for masking the cytokine within the PAC compound can enhance selectivity towards PD-1 expressing cells. By fine-tuning the binding affinity, these antibodies ensure that cytokine signaling is predominantly activated in the targeted cells, thereby maximizing therapeutic efficacy and minimizing off-target-cell effects.

*Example 5. Optimization of anti-PD-1 clone for IL-21 PAC*

The inventors developed two anti-PD-1 antibody clones 21A08Ap1 (heavy chain variable domain SEQ ID NO 009, LC variable domain SEQ ID NO 010) and XVT458-z2-m6 (heavy chain variable domain SEQ ID NO 011, LC variable domain SEQ ID NO 012). These antibodies do not interfere with the binding of existing checkpoint inhibitors (CPI) (Table 5). They may be used as targeting moieties in an IL-21 PAC, delivering the IL-21 signal directly to the appropriate PD-1+ CD8 T cells. This targeted delivery allows for optimal combination with existing anti-PD-1 therapeutics. The resulting PAC compounds provide highly targeted IL-21 signaling to PD-1 expressing cells and offer greater flexibility in combination with current CPI treatment regimens.

To confirm that the antibodies 21A08Ap1 and XVT458-z2-m6 are combinable with CPIs, binding was tested in presence of saturating concentrations of pembrolizumab and nivolumab by flow cytometry. Jurkat PD-1 expressing cells were incubated 30 minutes at 4°C with 10 µM of pembrolizumab and nivolumab. Without washing the cells antibodies 21A08Ap1 and XVT458-z2-m6 labelled with Alexa Fluor 647 were added at a fixed concentration of 100 nM. MFI levels of bound compound were compared to MFI in samples without competitor to determine the % signal inhibition. Inhibition was minimal, under 25%, showing neither pembrolizumab nor nivolumab significantly decreased the signal of antibodies 21A08Ap1 and XVT458-z2-m6 (Table 5).

Table 5. Competition of antibody clones 21A08Ap1 and XVT458-z2-m6 as human IgG1 on hPD-1 expressed on cells with commercial PD-1 antagonists, pembrolizumab and nivolumab. Flow cytometry % signal inhibition of the tested antibody using 100x molar excess of competitor

| Anti-PD-1 antibody clone | Signal inhibition in % with 100x molar excess checkpoint inhibitor antibody | |
|---|---|---|
| | pembrolizumab | nivolumab |
| XVT458-z2-m6 | 8.66 | 8.66 |
| 21A08Ap1 | 21.55 | 10.39 |

The inventors confirmed similar retention of PD-1 binding activity of constructs comprising a 21A081p1 cytokine PAC pre-incubated with Pembrolizumab, Nivolumab, Cemiplimab, Dostarlimab, Zeluvalimab, Tislelizumab, Ezabenlimab or Toripalimab (not shown). Off-target binding of both antibodies was excluded by testing cross-reactivity with the Retrogenix Cell Microarray platform.

Variants of 21A08Ap1 and XVT458-z2-m6 were generated as the inventors assayed various changes in the sequence with view to potential further improvements in parameters such as binding characteristics, manufacturability, stability, safety, transferability of animal study results to human patients and the like Table 6 and Table 7 show substitutions that were applied in the CDR regions or CDR flanking regions of the original 21A08Ap1 and XVT458-z2-m6 IgG antibodies, respectively. The selection of the amino acid substitution was crystal structure guided and based on physicochemical similarity, size of the amino acid side chain, exclusion of potential new PTM due to neighboring residues, as well as low predicted immunogenicity risk.

Table 6 Variants of anti-PD-1 antibody clone 21A08Ap1. Identifier and mutation are listed in the left column and the mutation compared to the indicated parental sequences.

| Anti-PD-1 clone name | Mutation | |
|---|---|---|
| | VL | VH |
| 21A08Ap1 | SEQ ID NO 010 | SEQ ID NO 009 |
| FHX509 | I98A | - |
| GDY271 | F35Y | - |
| GZZ692 | I49G | - |
| JOH712 | L40H | - |
| JZW468 | S95Q | - |
| KOW427 | L48G | - |
| KWH245 | W36T | - |
| KYC847 | S97T | - |
| NWL270 | I98N | - |
| SSR010 | I29V | - |

Table 7 Variants of anti-PD-1 antibody clone XVT458-z2-m6. Identifier and mutation are listed in the left column and the mutation compared to the indicated parental sequences.

| Anti-PD-1 clone name | Mutation | |
|---|---|---|
| | VL | VH |
| XVT458-z2-m6 | SEQ ID NO 012 | SEQ ID NO 011 |
| ACH836 | I29A | |
| BBJ602 | | A103G |
| DKR699 | | I57D |
| ICW519 | | K65Q |

(continued)

| Anti-PD-1 clone name | Mutation | |
|---|---|---|
| | VL | VH |
| JPK663 | I21A | |
| KOB135 | L54Q | |
| NRT742 | T51A | |
| OKM590 | | Y106V |
| PEV057 | | V93T |
| QGA408 | | Y60F |
| RZL961 | | N54Q |
| WAZ460 | S24R | |
| YCF391 | | I51T |
| ZTD928 | L95T | |

The antibody variable domains were expressed as human IgG1 antibodies by transient gene expression in CHO cells. Antibodies were purified from the supernatant using protein A. Expression levels in the supernatant, as well as yield and purity after protein A are reported in Table 8 and Table 9.

Table 8 Producibility parameters of parental 21A08Ap1 clone and of the variants as human IgG1. Parents and variant IgG1 antibodies were produced in 10 ml CHO cell cultures. The concentration of antibodies in the supernatant was determined by BLI using Gator® with a protein standard. After single step purification with protein A the concentration was measured, and the overall yield calculated. Purity of the compounds was determined by SDS-PAGE and SE-HPLC at 214 and 280 nm.

| Compound | Gator® (mg/L) | Concentrati on (mg/ml) | Yield (mg/L) | SDS-PAGE purity (%) | SE-HPLC purity |
|---|---|---|---|---|---|
| 21A08Ap1 | 667.26 | 1.31 | 365 | >95% | 100.0% |
| FHX509 | 442.22 | 2.72 | 170 | >95% | 99.40% |
| GDY271 | 292.61 | 1.79 | 139 | >95% | 98.75% |
| GZZ692 | 335.54 | 2.48 | 174 | >95% | 97.92% |
| JOH712 | 373.67 | 2.63 | 165 | >95% | 99.19% |
| JZW468 | 305.63 | 2.2 | 138 | >95% | 99.23% |
| KOW427 | 285.82 | 1.69 | 133 | >95% | 99.39% |
| KWH245 | 60.89 | 0.71 | 34 | >95% | 97.58% |
| KYC847 | 331.43 | 2.1 | 147 | >95% | 99.38% |
| NWL270 | 359.6 | 2.56 | 147 | >95% | 99.47% |
| SSR010 | 335.3 | 2.35 | 165 | >95% | 99.28% |

Table 9. Producibility parameters of parental XVT458-z2-m6 clone and of the indicated variants as human IgG1. The deimmunized variantsIgG1s were produced in 10 ml CHO cell cultures. The concentration of antibodies in the supernatant was determined by BLI using Gator® with a protein standard. After single step purification with protein A the concentration was measured, and the overall yield calculated. Purity of the compounds was determined by SDS-PAGE and SE-HPLC at 214 and 280 nm.

| Compound | Gator® (mg/L) | Concentration (mg/ml) | Yield (mg/L) | SDS-PAGE purity (%) | SE-HPLC purity |
|---|---|---|---|---|---|
| XVT458-z2-m6 | 379.82 | 2.87 | 215 | >95% | 97.84% |

(continued)

| Compound | Gator® (mg/L) | Concentration (mg/ml) | Yield (mg/L) | SDS-PAGE purity (%) | SE-HPLC purity |
|---|---|---|---|---|---|
| ACH836 | 295.11 | 2.29 | 149 | >95% | 98.79% |
| BBJ602 | 352.61 | 2.9 | 203 | >95% | 98.16% |
| DKR699 | 363.13 | 2.92 | 190 | >95% | 98.65% |
| ICW519 | 292.44 | 2.8 | 224 | >95% | 98.77% |
| JPK663 | 175.7 | 1.52 | 99 | >95% | 99.36% |
| KOB135 | 319.98 | 2.63 | 197 | >95% | 97.06% |
| NRT742 | 261.65 | 2.04 | 158 | >95% | 99.12% |
| OKM590 | 298.27 | 2.63 | 214 | >95% | 98.04% |
| PEV057 | 304.575 | 2.65 | 215 | >95% | 98.25% |
| QGA408 | 422.47 | 3.11 | 218 | >95% | 96.76% |
| RZL961 | 345.21 | 2.79 | 181 | >95% | 97.80% |
| WAZ460 | 306.41 | 2.61 | 202 | >95% | 97.98% |
| YCF391 | 390.47 | 2.84 | 178 | >95% | 97.85% |
| ZTD928 | 298.64 | 2.6 | 176 | >95% | 99.24% |

The binding kinetics of the anti-PD-1 antibodies to human and cynomolgus monkey PD-1 was assessed by SPR (Biacore T200, Cytiva). Anti-human IgG Fc (Cytiva Europe GmbH)) was immobilized on an amine reactive HLC1000M chip (XanTec Bioanalytics GmbH) using 1xHBSTE running buffer (XanTec Bioanalytics GmbH). The 10 ug/ml antibodies compounds were captured at a flow rate of 10 μl/min for 20 s. The antigens human and cynomolgus PD-1 (#PD1-H82E4 and #PD1-C52H5, respectively; AcroBiosystems AG) were diluted in 1xHBSTE running buffer (XanTec Bioanalytics GmbH) with 180 s association and 1800 s dissociation at flow rate 30 μl/min. Regeneration was carried out with 10 mM Glycine-HCl, pH 1.5 for 30 s and 3 M MgCl$_2$ pH 2.1 for 30 s. The association constant ($k_a$), dissociation rate constant ($k_d$) and equilibrium dissociation constant ($K_D$) are reported in Table 10 and Table 11.

Table 10 Kinetic binding parameters of parental clone 21A08Ap1 and variant compounds to human and cynomolgus PD-1.

| Anti-PD-1 clone | Binding to human PD-1 | | | Binding to cynomolgus monkey PD-1 | | |
|---|---|---|---|---|---|---|
| | $k_a$ (1/Ms) | $k_d$ (1/s) | $K_D$ (M) | $k_a$ (1/Ms) | $k_d$ (1/s) | $K_D$ (M) |
| 21A08Ap1 | 8.68E+04 | 1.10E-04 | 1.27E-09 | 2.06E+05 | 2.59E-04 | 1.25E-09 |
| FHX509 | 1.21E+05 | 8.38E-05 | 6.94E-10 | 3.41E+05 | 2.39E-04 | 6.99E-10 |
| GDY271 | 3.64E+05 | 1.30E-03 | 3.56E-09 | 1.91E+09 | 1.22E+01 | 6.38E-09 |
| GZZ692 | 1.48E+05 | 2.09E-04 | 1.41E-09 | 3.40E+05 | 1.53E-03 | 4.50E-09 |
| JOH712 | 1.63E+05 | 8.88E-05 | 5.46E-10 | 2.27E+05 | 2.52E-04 | 1.11E-09 |
| JZW468 | 1.46E+05 | 9.20E-05 | 6.31E-10 | 3.02E+05 | 2.90E-04 | 9.59E-10 |
| KOW427 | 1.36E+05 | 7.97E-05 | 5.86E-10 | 2.33E+05 | 2.96E-04 | 1.27E-09 |
| KWH245 | 1.67E+06 | 3.05E-04 | 1.83E-10 | 2.81E+05 | 6.44E-04 | 2.29E-09 |
| KYC847 | 1.44E+05 | 7.59E-05 | 5.28E-10 | 2.33E+05 | 1.39E-04 | 5.97E-10 |
| NWL270 | 4.29E+05 | 9.82E-05 | 2.29E-10 | 1.58E+05 | 1.50E-04 | 9.49E-10 |
| SSR010 | 2.60E+06 | 1.99E-04 | 7.67E-11 | 2.63E+05 | 3.01E-04 | 1.14E-09 |

Table 11 Kinetic binding parameters of parental clone XVT458-z2-m6 and variant compounds to human and cynomolgus PD-1. Nb = no binding.

| Anti-PD-1 clone | Binding to human PD-1 | | | Binding to cynomolgus monkey PD-1 | | |
|---|---|---|---|---|---|---|
| | $k_a$ (1/Ms) | $k_d$ (1/s) | $K_D$ (M) | $k_a$ (1/Ms) | $k_d$ (1/s) | $K_D$ (M) |
| XVT458-z2-m6 | 3.33E+05 | 1.74E-04 | 5.22E-10 | 4.99E+05 | 1.53E-04 | 3.06E-10 |
| ACH836 | 3.44E+05 | 1.17E-04 | 3.42E-10 | 4.93E+05 | 1.01E-04 | 2.05E-10 |
| BBJ602 | 1.54E+06 | 2.78E-02 | 1.81E-08 | 3.69E+08 | 3.15E+00 | 8.53E-09 |
| DKR699 | 2.00E+05 | 1.21E-04 | 6.06E-10 | 2.36E+05 | 2.03E-04 | 8.60E-10 |
| ICW519 | 3.65E+05 | 1.87E-04 | 5.11E-10 | 5.92E+05 | 1.76E-04 | 2.96E-10 |
| JPK663 | 3.36E+05 | 1.44E-04 | 4.29E-10 | 5.04E+05 | 1.24E-04 | 2.46E-10 |
| KOB135 | 3.29E+05 | 1.66E-04 | 5.06E-10 | 4.95E+05 | 1.79E-04 | 3.62E-10 |
| NRT742 | 4.26E+05 | 4.06E-04 | 9.55E-10 | 5.49E+05 | 2.97E-04 | 5.41E-10 |
| OKM590 | 4.09E+05 | 3.90E-04 | 9.55E-10 | 5.51E+05 | 3.45E-04 | 6.27E-10 |
| PEV057 | 3.63E+05 | 1.89E-04 | 5.21E-10 | 4.89E+05 | 1.60E-04 | 3.27E-10 |
| QGA408 | 4.34E+05 | 2.08E-04 | 4.80E-10 | 5.55E+05 | 1.82E-04 | 3.28E-10 |
| RZL961 | 4.44E+05 | 5.36E-04 | 1.21E-09 | 5.55E+05 | 4.36E-04 | 7.85E-10 |
| WAZ460 | 3.93E+05 | 2.02E-04 | 5.15E-10 | 4.85E+05 | 1.60E-04 | 3.31E-10 |
| YCF391 | 4.16E+05 | 3.36E-04 | 8.08E-10 | 3.91E+05 | 2.60E-04 | 6.64E-10 |
| ZTD928 | 3.49E+05 | 2.80E-04 | 8.01E-10 | 4.83E+05 | 2.43E-04 | 5.02E-10 |
| Alirocumab (neg. ctrl.) | Nb | | | Nb | | |

*Example 6. Design of PAC compounds*

Different strategies to fuse IL-21 (SEQ ID NO 015) to the anti-IL-21 antibody for generation of the cytokine arm of the PAC compounds, were assessed in order to find the PAC compounds with the best selectivity on PD-1+ cells. Fusion proteins of IL-21 C-terminus to the antibodies' N-terminus on the heavy chain or light chain were generated. In addition, IL-21 was embedded in the CDR regions of the antibody light chain by opening the VL peptide chain and fusing the open ends to the N- and C-termini of IL-21. In the following sections the cytokine arm fusion strategies will be described for various PAC compounds.

In patients receiving PD-1 checkpoint inhibitor (CPi) therapy with a PD-1 antagonist antibody, now a standard treatment for many cancers, the epitope targeted by blocking PD-1 targeted cytokine fusion proteins is already occupied by the CPi therapeutic. Thus, existing PD-1 targeted cytokines using blocking anti-PD-1 targeting antibodies have the drawback of being incompatible as an adjunct to standard checkpoint inhibition treatment regimes. In a first set of embodiments, as targeting arm of the PAC compounds, the anti-PD-1 antibody clones 21A08Ap1 (SEQ ID NO 013 heavy chain comprising variable domain SEQ ID NO 009, SEQ ID NO 014 light chain comprising variable domain SEQ ID NO 010) and XVT458-z2-m6 (HC variable domain SEQ ID NO 011, LC variable domain SEQ ID NO 012) were used. Each compound comprised an anti-PD-1 arm covalently associated via an IgG Fc portion (constant hu IgG1 HC SEQ ID NO 041) with an anti-IL-21 antibody in a bispecific antibody, heterotetrametric format, wherein either the HC or the LC of the anti-IL-21 antibody is linked to an IL-21 polypeptide. The two different HCs of the anti-PD-1 antibody and the anti-IL-21 antibody were engineered to form a heterodimer by inserting knobs-into-hole mutations (knobs: S354C, T366W; holes: Y349C, T366S, L368A, Y407V, by Kabat numbering). Correct assembly of the respective LCs was achieved without engineering of the natural sequences, by using one kappa LC (anti-IL-21 antibody) and one lambda (anti-PD-1 21A08Ap1 clone) chain. For constructs including the anti-PD-1 antibody clone family XVT458-z2-m6 (variable domains of HC, LC as provided in Table 7), correct pairing of the LC was insured by the presence of the IL-21 covalently linked to the VH or VL of the anti-IL-21 antibody arm or by LC engineering strategies (Immunoglobulin domain crossover, charged interface, etc.) (Klein (2018), Klein (2016), Bönisch (2017)). The Fc portion of the bispecific IgG1 compounds contains Fc silencing mutations L234A, L235A, P329A (Kabat numbering). In various embodiments, an IL-21 arm was provided based on variable domains of varying affinity inserted into the backbone of a ZAP122 antibody (HC SEQ ID NO 016, LC SEQ ID NO 017). An IL-21 polypeptide (SEQ ID NO 015) was covalently associated with either the HC or the LC via linkage arrangements as described in detail below. Similar PAC constructs could comprise muteins of IL-21, variants of the IL-21 polypeptide (SEQ ID NO 015) comprising at least one mutation, or chimeric IL-21 variants comprising deletions, insertions, or exchanges from other proteins, like IL-4 (Shen (2020), Bondensgaard (2007)).

*Heavy chain fusion protein*

Antibodies ZAP122, JSQ278, RPD212, IOO459, IFQ920 and EMV239 were used to generate fusion proteins to IL-21 on the antibodies' heavy chain. The N-terminus of the heavy chain was directly fused to the C-terminus of IL-21 through peptide linkers. Variants in linker length, flexibility and anti-IL-21 antibody affinity were generated as listed in (Table 10). The selectivity on PD-1+ cells was assessed in a cellular assay using Jurkat PD-1 expressing cells. Jurkat cells were pre-stimulated overnight in growth medium containing 0.01 pg/ml PMA and 1.0 pg/ml Ionomycin. Jurkat PD-1+ cells were washed twice with PBS. Half of the cells were incubated for 30 minutes with the parental anti-PD-1 IgG antibody clone 21A08Ap1, in order to block the PAC PD-1 binding site on the cells. Both the PD-1 blocked ant the non-blocked cells were then stimulated for 15 minutes with a dilution series of the PAC compounds. pSTAT3+ cells were detected by flow cytometry. The EC50 of the PAC compounds on blocked ($EC50_B$) or non-blocked ($EC50_{NB}$) Jurkat PD-1+ cells was calculated plotting the concentration against the %pSTAT3+ cells. The fold-difference in potency ($\Delta$) on blocked vs. non-blocked Jurkat PD-1 cells was calculated by dividing $EC50_B$ with $EC50_{NB}$.

Additionally, the binding of IL-21Rα to the PAC compounds was assessed by SPR (Biacore T200, Cytiva). The bispecific IL-21 comprising PAC compounds were captured on a human antibody capture chip (Xantec, HLC1000M; Cytiva, BR100839) at flow rate of 10 μl/ml to approximately 240-1600 RU. A serial dilution of hIL-21 Rα (AcroBiosystems, ILR-H5226) was used as analyte, diluted in HBSTE running buffer (Xantec) at a flow rate of 30 μl/min with 180s association and 600s of dissociation. A multi cycle analysis was performed using 5 different concentrations (1500, 474, 150, 47, and 15 nM) of the recombinant IL-21Rα. Regeneration was carried out with 3M $MgCl_2$ at pH=2.1 and Glycine-HCl at pH=1.5 at flow rates of 30 μL/min. The signal was considered positive for binding when 10% of the theoretical Rmax was achieved, calculated with following formula (Rmax: maximal signal, RL: captured ligand response (here PAC compounds), S: Stochiometry of captured ligand, MWA: molecular weight of analyte (here IL-21Rα; MWL: molecular weight of ligand):

$$Rmax = RL * S * \frac{MWA}{MWL}$$

PAC compounds featuring a cytokine arm with IL-21 fused to the N-terminus of the anti-IL-21 antibody heavy chain were generated using varying linkers and different anti-IL-21 antibody variants. All PAC compounds listed in Table 12 exhibit enhanced potency in inducing pSTAT3 signalling when PD-1 was accessible for binding, indicating effective masking of the IL-21 receptor binding site in a non-cell bound state, and effective unmasking and potent IL-21R triggering in the target cell bound state.

Table 12. PAC compounds with anti-IL-21 antibody variants, with HC linkage. For all constructs the anti-PD-1 antibody clone 21A08Ap1 was used. Potency in inducing STAT3 phosphorylation in Jurkat PD-1 cells blocked ($EC50_B$)/non-blocked ($EC50_{NB}$), the difference ($\Delta$) and binding of the rIL-21Rα by SPR. * average of n=2 experiments *average of n=3 experiments. Standard deviation in parenthesis. Nt = not tested; § positive based on calculation, however subjective review of sensorgrams does not indicate binding

| Compound name | Anti-IL-21 antibody | Linker to IL-21 SEQ ID NO | Potency on Jurkat PD-1 cells | | | IL-21Rα at binding concentration (nM) | | | | |
|---|---|---|---|---|---|---|---|---|---|---|
| | | | $EC50_{NB}$ (nM) | $EC50_B$ (nM) | $\Delta$ | 15 | 47 | 150 | 474 | 1500 |
| rhIL-21 | | | 0.0006* (0.0004) | 0.0008* (0.0006) | 1.050* (0.050) | no | yes | yes | yes | yes |
| DHJ369 | ZAP122 | 024 | 0.24 | 2.56 | 10.7 | nt | nt | nt | nt | nt |
| JKJ112 | ZAP122 | 025 | 0.52 | 17.18 | 33.20 | nt | nt | nt | nt | nt |
| ZBA121 | ZAP122 | 026 | 0.86 | 102.60 | 119.19 | nt | nt | nt | nt | nt |
| DMT806 | ZAP122 | 027 | 0.17* (0.19) | 12.39* (10.40) | 134.34* (56.31) | no | no | no | no§ | yes |
| IFM656 | JSQ278 | 027 | 0.21 * (0.18) | 15.86* (5.93) | 112.15* (45.98) | no | no | no | no | no |

(continued)

| Compound name | Anti-IL-21 antibody | Linker to IL-21 SEQ ID NO | Potency on Jurkat PD-1 cells | | | IL-21Rα at binding concentration (nM) | | | | |
|---|---|---|---|---|---|---|---|---|---|---|
| | | | $EC50_{NB}$ (nM) | $EC50_B$ (nM) | $\Delta$ | 15 | 47 | 150 | 474 | 1500 |
| SUR250 | RPD212 | 027 | 0.08* (0.069) | 5.36* (3.90) | 68.75* (11.88) | no | no | no | yes | yes |
| CMQ332 | IOO459 | 026 | 0.012# (0.004) | 0.034# (0.008) | 2.86# (0.24) | nt | nt | nt | nt | nt |
| OUQ507 | IOO459 | 025 | 0.024 | 0.42 | 17.50 | nt | nt | nt | nt | nt |
| JAK529 | IFQ920 | 026 | 0.027# (0.016) | 0.44# (0.040) | 26.56# (17.04) | nt | nt | nt | nt | nt |
| EXX354 | IFQ920 | 025 | 0.039# (0.025) | 0.28# (0.135) | 8.58# (2.12) | nt | nt | nt | nt | nt |
| BKC311 | EMV239 | 025 | 0.0031 | 0.0011 | 0.35 | yes | yes | yes | yes | yes |
| XIN673 | EMV239 | 026 | 0.0031 | 0.0036 | 1.16 | nt | nt | nt | nt | nt |

*Light chain fusion proteins*

Antibodies JSQ278, RPD212, ZAP122m3, ZXI489, IFQ920 and IOO459 were used to generate fusion proteins to IL-21 on the antibodies' light chain. The N-terminus of the light chain was directly fused to the C-terminus of IL-21 through peptide linkers. Variants of the anti-IL-21 antibody were generated as listed in Table 13. The selectivity for PD-1+ cells was evaluated by assessing pSTAT3+ cells in Jurkat PD-1+ cells, as previously described.

Table 13 PAC compounds with anti-IL-21 antibody variants, with LC linkage. For all constructs the anti-PD-1 antibody clone 21A08Ap1 was used. Potency in inducing STAT3 phosphorylation in Jurkat PD-1 cells blocked ($EC50_B$)/non-blocked ($EC50_{NB}$), the difference ($\Delta$) and binding of the rIL-21Rα by SPR. EC50 average of 3 or 2(*) experiments. Standard deviation in parenthesis. nt = not tested; § positive based on calculation, however subjective review of SPR sensorgram does not indicate binding.

| Compound name | Anti-IL-21 antibody | Linker to IL-21 (Seq ID NO) | Potency on Jurkat PD-1 cells | | | IL-21Rα binding at concentration (nM) | | | | |
|---|---|---|---|---|---|---|---|---|---|---|
| | | | $EC50_{NB}$ (nM) | $EC50_B$ (nM) | $\Delta$ | 15 | 47 | 150 | 474 | 1500 |
| rhIL-21 | | | 0.0006 (0.0004 ) | 0.0008 (0.0006 ) | 1.050 (0.050) | no | yes | yes | yes | yes |
| CHU368 | JSQ278 | 028 | 0.583 (0.232) | 2.16 (1.534) | 3.3 (1.1) | no | no | no | no | yes |
| ECW089 | JSQ278 | 030 | 0.0963* (0.007) | 0.13* (0.048) | 1.3* (0.4) | nt | nt | nt | nt | nt |
| EDV817 | JSQ278 | 027 | 0.0969* (0.005) | 0.13* (0.042) | 1.3* (0.4) | nt | nt | nt | nt | nt |
| EFY288 | JSQ278 | 025 | 0.129* (0.004) | 0.234* (0.056) | 1.8* (0.4) | nt | nt | nt | nt | nt |
| XOW683 | RPD212 | 028 | 0.413 (0.193) | 1.836 (1.185) | 4.2 (1.3) | no | no | no | no | no |
| GWM570 | ZAP122m3 | 028 | 0.257 (0.182) | 2.127 (1.091) | 12.4 (8.8) | no | no | no | no | yes |

(continued)

| Compound name | Anti-IL-21 antibody | Linker to IL-21 (Seq ID NO) | Potency on Jurkat PD-1 cells | | | IL-21Rα binding at concentration (nM) | | | | |
|---|---|---|---|---|---|---|---|---|---|---|
| | | | EC50$_{NB}$ (nM) | EC50$_B$ (nM) | Δ | 15 | 47 | 150 | 474 | 1500 |
| CDP278 | ZAP122 m3 | 030 | 0.167* (0.058) | 1.053* (0.117) | 7.4* (3.3) | nt | nt | nt | nt | nt |
| CDP451 | ZAP122 m3 | 027 | 0.106* (0.048) | 1.244* (0.019) | 14.7* (6.501) | nt | nt | nt | nt | nt |
| EJD551 | ZXI489 | 031 | 0.016 (0.003) | 1.063 (0.028) | 69.7 (14.7) | no | no | no | no | no |
| ETR048 | ZXI489 | 028 | 0.012* (0.002) | 0.751* (0.044) | 63.9* (14.7) | nt | nt | nt | nt | nt |
| GMH764 | ZXI489 | 030 | 0.013* (0.002) | 0.872* (0.153) | 66.324* (2.6) | nt | nt | nt | nt | nt |
| GSV075 | ZXI489 | 027 | 0.023* (0.020) | 0.627* (0.180) | 37.5* (24.5) | nt | nt | nt | nt | nt |
| ABD523 | IFQ459 | 031 | 0.013* (0.002) | 0.688* (0.182) | 58.1* (24.6) | nt | nt | nt | nt | nt |
| YCV364 | IFQ920 | 028 | 0.009* (0.001) | 1.332* (0.127) | 148.7* (33.7) | no | no | yes | yes | yes |
| BBU753 | IFQ920 | 030 | 0.015* (0.004) | 0.312* (0.12) | 24.7* (15.0) | nt | nt | nt | nt | nt |
| PAP722 | IOO459 | 028 | 0.019 (0.019) | 1.967 (1.522) | 371.3 (247) | no | no | no | no | yes |

All PAC compounds listed in Table 13 exhibit enhanced potency in inducing pSTAT3 signalling when PD-1 was accessible for binding, indicating effective masking of the IL-21 receptor binding site in a non-cell bound state, and effective unmasking and potent IL-21R triggering in the target cell bound state.

*Embedded in CDR-L1*

Based on the crystal structure of the anti-IL-21 Fab/IL-21 complex, the distances from the N-terminus (Q30) and C-terminus (S162) of IL-21 to the amino acids in the CDR-L1 region of the antibody were measured (Table 14). The amino acids closest to both termini of IL-21 in the non-covalently associated cytokine and antibody were selected for embedding IL-21 within the antibody. The antibody light chain was cleaved between the CDR-L1 residues Q27 and G28, as numbered compared to the SEQ ID NO 004 parent sequence in variant antibodies. The N-terminus of IL-21 (Q30) was fused to the antibody light chain at Q27 through glycine-serine linkers of various length, and the C-terminus of IL-21 (S162) was fused to the antibody light chain at G28, using a glycine-serine linker of the same length used at the N-terminus. This process effectively embedded IL-21 within the CDR-L1 sequence of the antibody. Using this strategy, IL-21 was embedded in the antibodies JSQ278, RPD212, ZAP122m3, IFQ920, ZXI489 and IOO459. The selectivity for PD-1+ cells was evaluated by assessing pSTAT3+ cells in Jurkat PD-1+ cells, as previously described (Table 15). Binding of the IL-21Rα to the PAC fused IL-21 was assessed by SPR, as described above.

Table 14 Distance of IL-21 to CDRL1 amino acids in Å. Q27 and G28 of the CDR-L1 were chosen for the embedding of IL-21.

| CDR-L1 Fab | IL-21 | |
|---|---|---|
| | N-ter Q30 | C-ter S162 |
| R24 | 15.2 | 20.6 |

(continued)

| CDR-L1 Fab | IL-21 | |
| --- | --- | --- |
| | N-ter Q30 | C-ter S162 |
| A25 | 12.8 | 17.7 |
| S26 | 14.4 | 14.3 |
| **Q27** | **10.9** | **10.9** |
| **G28** | **8.4** | **13.5** |
| I29 | 7.3 | 15.6 |
| S30 | 4.3 | 16.7 |
| S31 | 5.3 | 19.7 |
| W32 | 8.1 | 17.9 |
| L33 | 11.1 | 22.6 |
| A34 | 14.4 | 25.4 |

Table 15 PAC compounds with various anti-IL-21 antibody variants and IL-21 embedded in CDR-L1 of anti-IL-21 antibody using various linker lengths. Potency in inducing STAT3 phosphorylation in Jurkat PD-1 cells blocked (EC50$_B$)/non-blocked (EC50$_{NB}$), the difference ($\Delta$) and binding of the rIL-21R$\alpha$ by SPR.EC50 average of 3 experiments, or* 2 experiments. ForAXV578 the values are from 1 experiment (#).Standard deviation in parenthesis. nt = not tested; § positive based on calculation, however subjective review of SPR sensorgram does not indicate binding; &negative based on calculation, however subjective review of SPR sensorgram does indicate binding.

| Compound name | Anti-IL-21 antibody | Linker to IL-21 | Anti-PD-1 antibody | Potency on Jurkat PD-1 cells | | | IL-21R$\alpha$ binding at concentration (nM) | | | | |
| --- | --- | --- | --- | --- | --- | --- | --- | --- | --- | --- | --- |
| | | | | EC50$_{NB}$ (nM) | EC50$_B$ (nM) | $\Delta$ | 1 5 | 47 | 15 0 | 47 4 | 1500 |
| rhIL-21 | | | | 0.0006 (0.0004) | 0.0008 (0.0006) | 1.050 (0.05 0) | n o | ye s | ye s | ye s | yes |
| JAA693 | JSQ278 | 029 | 21A 08A p1 | 0.291 (0.027) | 0.90 (0.13) | 3.1 (0.16) | n o | no | no | no | no§ |
| JNR076 | JSQ278 | 032 | 21A 08A p1 | 0.455* (0.103) | 0.908* (0.139) | 2.0* (0.2) | nt | nt | nt | nt | nt |
| JQC456 | JSQ278 | 033 | 21A 08A p1 | 0.487* (0.237) | 0.545* (0.007) | 1.5* (0.7) | nt | nt | nt | nt | nt |
| KCD869 | JSQ278 | 030 | 21A 08A p1 | 0.131* (0.044) | 0.288* (0.108) | 2.2* (0.1) | nt | nt | nt | nt | nt |
| YAJ975 | RPD212 | 029 | 21A 08A p1 | 0.174 (0.074) | 1.27 (0.911) | 6.4 (2.5) | n o | no | no | no | no |
| IGQ626 | ZAP122 m3 | 034 | 21A 08A p1 | 0.004* (0.001) | 0.655* (0.138) | 170.9 * (4.4) | nt | nt | nt | nt | nt |
| DJC951 | ZAP122 m3 | 029 | 21A 08A p1 | 0.005* (0.002) | 1.07* (0.945) | 155.6 * (123) | n o | no | no | ye s | yes |

(continued)

| Compound name | Anti-IL-21 antibody | Linker to IL-21 | Anti-PD-1 antibody | Potency on Jurkat PD-1 cells | | | IL-21Rα binding at concentration (nM) | | | | |
|---|---|---|---|---|---|---|---|---|---|---|---|
| | | | | EC50NB (nM) | EC50B (nM) | Δ | 1 5 | 47 | 15 0 | 47 4 | 1500 |
| IZN036 | ZAP122m3 | 032 | 21A08Ap1 | 0.011 * (0.002) | 0.799* (0.017) | 78.9* (17.4) | nt | nt | nt | nt | nt |
| JCH768 | ZAP122m3 | 033 | 21A08Ap1 | 0.006* (0.0003) | 0.374* (0.081) | 62.1 * (16.4) | nt | nt | nt | nt | nt |
| KKY261 | ZXI489 | 034 | 21A08Ap1 | 0.003 (0.002) | 2.244 (0.835) | 992.5 (533) | nt | nt | nt | nt | nt |
| LLX102 | ZXI489 | 029 | 21A08Ap1 | 0.006 (0.003) | 3.237 (1.062) | 665.2 (173) | n o | no | no | ye s& | yes |
| LPZ870 | ZXI489 | 032 | 21A08Ap1 | 0.009* (0.005) | 2.442* (0.736) | 317.2 * (99.7) | nt | nt | nt | nt | nt |
| NFQ601 | ZXI489 | 033 | 21A08Ap1 | 0.014* (0.008) | 2.626* (1.096) | 209.8 * (35.9) | nt | nt | nt | nt | nt |
| HMZ098 | IFQ459 | 034 | 21A08Ap1 | 0.008* (0.003) | 0.798* (0.151) | 107.1 * (22.8) | nt | nt | nt | nt | nt |
| WIX772 | IFQ920 | 029 | 21A08Ap1 | 0.009 (0.005) | 3.50 (2.076) | 486.0 (194) | n o | no | ye s | ye s | yes |
| HZB944 | IFQ459 | 032 | 21A08Ap1 | 0.016* (0.0001) | 0.886* (0.319) | 55.1* (20.0) | nt | nt | nt | nt | nt |
| NXX045 | IOO459 | 029 | 21A08Ap1 | 0.007* (0.005) | 1.39* (1.094) | 111.3 * (60.4) | n o | no | ye s | ye s | yes |
| AXV578 | IFQ920 | 029 | non-binder | 10.56# | 15.50# | 1.47# | n o | no | no | ye s& | yes& |

All PAC compounds listed in Table 15 exhibit enhanced potency in inducing pSTAT3 signaling when PD-1 was accessible for binding, indicating effective masking of the IL-21 receptor binding site in a non-cell bound state, and effective unmasking and potent IL-21R triggering in the target cell bound state.

*Embedded in CDR-L3*

Similarly, embedding of IL-21 in the CDR-L3 was performed at the closest residues (Table 16) to the N-terminus (Q30) and C-terminus (S162) of IL-21 through insertion between N92 and S93 numbered relative to SEQ ID NO 004. Fusion proteins with IL-21 embedded in the CDRL-3 were generated using the anti-IL21 antibodiesJSQ278, RPD212, ZAP122m3, ZXI489 and IOO459 with different linker lengths. The selectivity for PD-1+ cells was evaluated by assessing pSTAT3+ cells in Jurkat PD-1+ cells, as previously described (Table 17). Binding of the IL-21Rα to the PAC fused IL-21 was assessed by SPR, as described above.

Table 16 Distance of IL-21 to CDRL3 amino acids Å. N92 and S93 of the CDR-L1 were chosen for the embedding of IL-21.

| CDR-L3 Fab | IL-21 | |
|---|---|---|
| | N-ter Q30 | C-ter S162 |
| Q89 | 15.3 | 23.4 |
| Q90 | 13.9 | 17.6 |
| A91 | 12.8 | 18.7 |
| **N92** | **8.8** | **13.3** |
| **S93** | **13.4** | **13.3** |
| F94 | 17.0 | 16.0 |
| P95 | 18.6 | 17.3 |
| L96 | 17.8 | 20.5 |
| T97 | 18.1 | 19.9 |

Table 17 PAC compound with IL-21 embedded in CDR-L3 of anti-IL-21 antibody. Potency in inducing STAT3 phosphorylation in Jurkat PD-1 cells blocked (EC50$_B$)/non-blocked (EC50$_{NB}$)and binding of the rIL-21R$\alpha$ by SPR. EC50 average of 3 or (*) 2 experiments. For VYL308, QGQ134 and DKG223 the values are from 1 experiment (#). Standard deviation in parenthesis. nt = not tested; § positive based on calculation, however subjective review of SPR sensorgram does not indicate binding; &negative based on calculation, however subjective review of SPR sensorgram does indicate binding.

| Name | Anti-IL-21 antib ody | Linke r to IL-21 | Anti-PD-1 antibo dy | Potency on Jurkat PD-1 cells | | | IL-21R$\alpha$ binding at concentration (nM) | | | | |
|---|---|---|---|---|---|---|---|---|---|---|---|
| | | | | EC50$_{NB}$ (nM) | EC50$_B$ (nM) | Δ | 15 | 47 | 150 | 474 | 150 0 |
| rhIL-21 | - | - | - | 0.0006 (0.0004) | 0.0008 (0.000 6) | 1.050 (0.050) | no | yes | yes | yes | yes |
| VYL308 | JSQ 278 | No linker | 21A08 Ap1 | 0.0007# | 0.056# | 79.9# | yes | yes | yes | yes | yes |
| SHZ400 | JSQ 278 | 034 | 21A08 Ap1 | 0.002* (0.001) | 3.447* (0.799) | 1906* (983) | no | no | no | yes & | yes & |
| TVU155 | JSQ 278 | 029 | 21A08 Ap1 | 0.004 (0.002) | 0.951 (0.100) | 292.9 (161) | no | no | no | no | no |
| ULS916 | JSQ 278 | 032 | 21A08 Ap1 | 0.006* (0.003) | 0.873* (0.092 5) | 190.9* (75.4) | no | no | no | yes | yes |
| UNY098 | JSQ 278 | 033 | 21A08 Ap1 | 0.007* (0.005) | 0.333* (0.105) | 81.7* (45.7) | no | no | no | yes | yes |
| YWW79 8 | RPD 212 | 029 | 21A08 Ap1 | 0.004 (0.002) | 0.526 (0.043) | 191.0 (119.05) | no | no | yes | yes | yes |
| PFV543 | ZAP 122 m3 | 034 | 21A08 Ap1 | 0.001* (0.001) | 0.680* (0.174) | 1266* (746) | nt | nt | nt | nt | nt |
| QJE251 | ZAP 122 m3 | 029 | 21A08 Ap1 | 0.003* (0.002) | 0.418* (0.097) | 347.5* (245) | nt | nt | nt | nt | nt |
| QTI591 | ZAP 122 m3 | 032 | 21A08 Ap1 | 0.002* (0.002) | 0.289* (0.109) | 325.0* (209) | nt | nt | nt | nt | nt |
| SCH932 | ZAP 122 m3 | 033 | 21A08 Ap1 | 0.003* (0.002) | 0.163* (0.023) | 142.6* (104) | nt | nt | nt | nt | nt |

(continued)

| Name | Anti-IL-21 antibody | Linker to IL-21 | Anti-PD-1 antibody | Potency on Jurkat PD-1 cells | | | IL-21Rα binding at concentration (nM) | | | | |
|---|---|---|---|---|---|---|---|---|---|---|---|
| | | | | EC50$_{NB}$ (nM) | EC50$_B$ (nM) | Δ | 15 | 47 | 150 | 474 | 150 0 |
| VUB931 | ZXI4 89 | 029 | 21A08 Ap1 | 0.005* (0.003) | 1.484* (0.061) | 464.1* (270.2) | nt | nt | nt | nt | nt |
| WBL338 | ZXI4 89 | 032 | 21A08 Ap1 | 0.003 (0.003) | 1.525 (0.427) | 808.6 (465.3) | no | no | no | yes | yes |
| OMU27 6 | IOO4 59 | 029 | 21A08 Ap1 | 0.007* (0.003) | 4.612* (1.450) | 715.4* (86.6) | nt | nt | nt | nt | nt |
| DKG223 | JSQ 278 | 034 | XVT45 8-z2-m6 | 0.0079# | 5.36# | 678.2# | no | no | no | yes & | yes & |
| QGQ13 4 | JSQ 278 | 034 | non-binder | 5.409# | 13.40# | 2,5# | no | no | no | yes | yes & |

*Embedded in CDR-L2*

Fusion proteins with IL-21 embedded between the CDR-L2 amino acids Q55 and S56 were generated using linkers of increasing lengths, ranging from 5 to 15 amino acids (Table 18). Binding of the IL-21Rα to the IL-21 PAC was assessed by SPR, as described above.

Table 18 PAC compounds with one anti-IL-21 antibody variant and IL-21 embedded in CDR-L2 of the anti-IL-21 antibody through various linkers. For all constructs the anti-PD-1 antibody clone 21A08Ap1 was used. Potency in inducing STAT3 phosphorylation in Jurkat PD-1 cells blocked (EC50$_B$)/non-blocked (EC50$_{NB}$), the difference (Δ) and binding of the rIL-21Rα by SPR. EC50 Average of 3 experiments, or * 2 experiments. Standard deviation in parenthesis.

| Name | Anti-IL-21 Ab | Linker to IL-21 | Potency on Jurkat PD-1 cells | | | IL-21Rα binding at concentration (nM) | | | | |
|---|---|---|---|---|---|---|---|---|---|---|
| | | | EC50$_{NB}$ (nM) | EC50$_B$ (nM) | Δ | 15 | 47 | 150 | 474 | 1500 |
| rhIL-21 | | | 0.0006 (0.0004) | 0.0008 (0.000 6) | 1.050 (0.050) | no | yes | yes | yes | yes |
| GOP758 | ZAP1 22 | 028 | 0.227 (0.292) | 0.51 (0.48) | 7.2 (5.45) | no | no | no | yes | yes |
| VCG677 | ZAP1 22 | 030 | 0.039 (0.050) | 2.30 (2.41) | 142.6 (79.02) | no | no | yes | yes | yes |
| ZNP367 | ZAP1 22 | 027 | 0.036* (0.034) | 0.97* (0.73) | 73.1 * (50.27) | no | no | no | yes | yes |

*Valency variants*

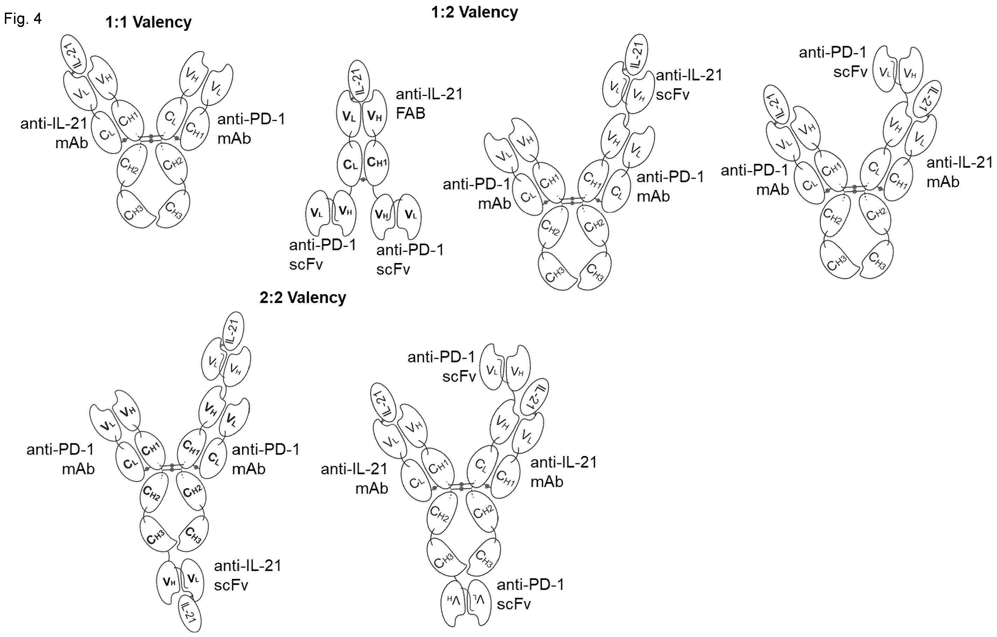

Different valency variants may be used to increase the avidity effect of either the targeting arm or the potency of the cytokine arm of a PAC compound. Incorporating varying numbers of anti-PD-1 or anti-IL-21 binding moieties may also enhance selectivity, reducing off-target effects, and fine-tune the potency for more robust therapeutic effects at lower doses. To achieve different valency, single-chain variable fragments (scFv), antigen-binding fragments (Fabs), or other formats (e.g. diabodies, single-chain diabodies, VHH etc.) targeting PD-1 or IL-21 with IL-21 fusion can be fused to the N- or C-terminus of PD-1 or IL-21 targeting homodimers, or PD-1 and IL-21 targeting heterodimers. 1:2, 2:1, and 2:2 valency may be obtained with these designs (Figure 4).

Examples of valency variants were developed, incorporating either one or two anti-PD-1 binding moieties or one or two anti-IL-21 binding moieties (JSQ278 or RPD212 in scFv format), covalently linked to IL-21. IL-21 heavy chain (HC),light chain (LC) fusions or CDR-L3 embedded variants were tested, with fusions occurring at either the N-terminus (N-ter) or the C-terminus (C-ter) of the molecules (Table 19) selectivity for PD-1+ cells was evaluated by assessing pSTAT3+ cells in Jurkat PD-1+ cells, as previously described. Furthermore, the binding of IL-21Rα to the PAC fused IL-21 was assessed by SPR, as described above.

Table 19 PAC compounds with 1:2 or 2:1 valency. Orientation of scFv from N-ter to C-ter. Potency in inducing STAT3 phosphorylation in Jurkat PD-1 cells blocked (EC50$_B$)/non-blocked (EC50$_{NB}$), the difference (Δ) and binding of the rIL-21Rα by SPR. EC50 average of 3 experiments, with standard deviation in parenthesis for QUG430 and VQX680. EC50 values from 1 experiment for NYX587, QUP911, UPH584, ZVV304. nt = not tested; § positive based on calculation, however subjective review of SPR sensorgram does not indicate binding; &negative based on calculation, however subjective review of SPR sensorgram does indicate binding.

| Name | Anti-IL-21 Ab | Anti-PD-1 antibody | scFv orientation | # of moieties | | IL-21 Fused/em bedded | Linker to IL-21 |
|---|---|---|---|---|---|---|---|
| | | | | anti PD1 | anti IL-21 | | |
| NYX58 7 | JSQ278sc Fv | XVT458-z2-m6 | VH-VL | 2 | 1 | CDRL3 | 034 |
| QUP9 11 | JSQ278sc Fv | XVT458-z2-m6 | VL-VH | 2 | 1 | CDR-L3 | 034 |
| UPH58 4 | JSQ278sc Fv | 21A08Ap1 | VH-VL | 2 | 1 | CDR-L3 | 034 |
| ZW30 4 | JSQ278sc Fv | 21A08Ap1 | VL-VH | 2 | 1 | CDR-L3 | 034 |
| QUG4 30 | RPD212sc Fv | 21A08Ap1 | VL-VH | 2 | 1 | N-ter | 028 |
| VQX68 0 | RPD212 | 21A08Ap1s cFv | VL-VH | 1 | 2 | N-ter | 028 |

Table 19 (continued), further columns

| | Potency on Jurkat PD-1 cells | | | KD of IL-21Rα [nM] | | | | |
|---|---|---|---|---|---|---|---|---|
| Name | EC50 NB (nM) | EC50 B (nM) | Δ | 15 | 47 | 150 | 474 | 1500 |
| NYX587 | 0.0018 | 3.38 | 1873.2 | no | no | no | yes | yes |
| QUP911 | 0.0012 | 3.26 | 2644.2 | no | no | no | yes$^&$ | yes |
| UPH584 | 0.0064 | 3.24 | 508.9 | no | no | yes$^&$ | yes | yes |
| ZW304 | 0.0033 | 3.47 | 1064.7 | no | no | no | yes$^&$ | yes |
| QUG430 | 0.69 (0.18) | 10.48 (11.90) | 13.8 (14.3) | no | no | no | no | no |
| VQX680 | 0.37 (0.32) | 1.43 (1.26) | 4.3 (1.4) | no | no | no | no | yes |

The difference in potency of all tested valency variant compounds (Δ) on PD-1+ cells (EC50$_{NB}$) and PD-1 blocked cells (EC50$_B$) indicates successful cytokine unmasking and signaling of the PACs when bound to PD-1 on the cell surface.

*Example 7: PACs with PD-1 blocking antibodies*

A PD-1 blocking antibody (i.e. blocking PD-1/PD-L1 interaction) can be effectively utilized in a Proximity Activated Cytokine (PAC) compound to enhance anti-tumor immune responses. By blocking the PD-1/PD-L1 interaction, the antibody can prevent the inhibitory signaling that typically dampens T cell activity, thereby reinvigorating PD-1-expressing cells. When used in a PAC, the PD-1 blocking antibody not only targets the compound to PD-1 expressing cells but also amplifies the therapeutic effect by concurrently inhibiting the checkpoint pathway. PD-1 blocking antibodies such as pembrolizumab (LC variable domain SEQ ID NO 020, HC variable domain SEQ ID NO 021), nivolumab (LC variable domain SEQ ID NO 022, HC variable domain 023), prolgolimab (LC variable domain SEQ ID NO 036, HC variable domain SEQ ID NO 035), zimberelimab (LC variable domain SEQ ID NO 038, HC variable domain SEQ ID NO 037) or of AMG256 (LC variable domain SEQ ID NO 040, HC variable domain SEQ ID NO 039) may be used as the antigen binding domain of the targeting arm and combined to cytokine arms described in Table 12, 13, 15, or 17. IL-21 PAC compounds with prolgolimab (LC variable domain SEQ ID NO 036, HC variable domain SEQ ID NO 035), zimberelimab (LC variable domain SEQ ID NO 038, HC variable domain SEQ ID NO 037) or of AMG256 (LC variable domain SEQ ID NO 040, HC variable domain SEQ ID NO 039) as PD-1 binding antibodies were generated as bispecific antibody-IL-21 fusion proteins with the WBL338 orSHZ400 anti-IL-21/IL-21 arms (Table 20). The bispecific compounds were generated by inserting knobs-into-hole mutations (knobs: S354C, T366W; holes: Y349C, T366S, L368A, Y407V, by Kabat numbering) in the constant domains of the heavy chains. Correct assembly of the respective LCs was achieved without engineering of the natural sequences, by using one kappa LC (anti-IL-21 antibody) and one lambda (anti-PD-1) chain.

Table 20 PAC compounds with various PD-1 blocking mAbs and IL-21 antibody/IL-21 fusion moieties as indicated.

| Name | Anti-IL-21 Ab | Anti-PD-1 antibody | IL-21 Fused/embedded | Linker to IL-21 |
|------|---------------|--------------------|-----------------------|-----------------|
| BQD985 | JSQ278 | prolgolimab | CDR-L3 | 034 |
| EPL638 | JSQ278 | zimberelimab | CDR-L3 | 034 |
| QSA863 | ZXI489 | prolgolimab | CDR-L3 | 032 |
| YJI844 | ZXI489 | zimberelimab | CDR-L3 | 032 |
| HGC721 | JSQ278 | AMG256 | CDR-L3 | 034 |
| OXA105 | ZXI489 | AMG256 | CDR-L3 | 032 |

*Example 8. Potency of IL-21 PAC compounds on human PBMCs*

IL-21R is broadly expressed by immune cells including CD4 and CD8 T cells, NK cells, B cells, dendritic cells, and macrophages such that recombinant cytokines may have undesired immunological effects. PACs according to the invention preferentially provide IL-21 signal to exhausted T cells expressing PD-1. Blockade of the PD-1 binding site by parent anti-PD-1 antibody prior to subsequent administration of the PAC to the assay was performed to determine how STAT3 signaling is influenced when delivered by means of a PAC comprising a cell targeting feature in addition to rhIL-21. Representative compounds PAP722, YCV364, NXX045, WIX772, KKY261, LLX102, VUB931, WBL338, DJC951, YWW798, SHZ400, TVU155, DMT806 were tested for their potency in inducing selective STAT3 phosphorylation on PD-1+ CD8 T cells *in vitro*. Frozen PBMCs were thawed and pre-activated with anti-CD3 and anti-CD28 antibodies. Post activation, half of the cells were incubated for 30 minutes with the parental anti-PD-1 IgG antibody clone 21A08Ap1, in order to block the PAC PD-1 binding site on the cells. Both the PD-1 blocked and the non-blocked cells were treated with IL-21 PAC compounds at increasing concentrations. Cells were stained with anti CD25, CD3, CD4, CD8, CD56, PD-1, FoxP3, and pSTAT3 antibodies for flow cytometry, along with a live/dead marker. Following staining, cells were analyzed by flow cytometry to quantify pSTAT3+ cells. The EC50 of the PAC compounds on blocked ($EC50_B$) or non-blocked ($EC50_{NB}$) CD8+PD-1+ cells was calculated by plotting the concentration against the %pSTAT3+ cells. The fold-difference in potency ($\Delta$) on blocked vs. non-blocked CD8+PD-1+ cells was calculated by dividing $EC50_{NB}$ with $EC50_B$ (Table 21).

The potency of IL-21 signaling was significantly reduced when PD-1 targeting was prevented, an effect not observed with rhIL-21, indicating that the PAC compounds effectively minimize off-target IL-21 signaling in PD-1 negative cells. This targeted approach ensures that PACs according to the invention preferentially induce IL-21 signaling in target cells that express PD-1.

Table 21 Potency of representative IL-21 PACs in inducing phosphorylation of STAT3 in CD8+PD-1+ blocked cells ($EC50_B$) or non-blocked cells ($EC50_{NB}$), difference ($\Delta$), n= PBMCs from 2 donors.

| Compound | $EC50_{NB}$ on CD8+PD-1+ cells | $EC50_B$ on CD8+PD-1+ cells | $\Delta$ |
|----------|-------------------------------|-----------------------------|----------|
| rec hIL21 | 0.0018 | 0.0026 | 1.44 |
| PAP722 | 0.69 | 2.96 | 4.30 |
| YCV364 | 0.71 | 2.98 | 4.21 |
| DJC951 | 0.33 | 2.03 | 6.06 |
| NXX045 | 0.37 | 4.93 | 13.36 |
| WIX772 | 0.83 | 5.76 | 6.95 |
| KKY261 | 1.07 | 3.94 | 3.70 |
| LLX102 | 0.70 | 4.21 | 6.06 |
| VUB931 | 0.36 | 2.07 | 5.79 |
| WBL338 | 0.47 | 3.82 | 8.18 |
| YWW798 | 0.53 | 1.82 | 3.45 |

(continued)

| Compound | EC50$_{NB}$ on CD8+PD-1+ cells | EC50$_{B}$ on CD8+PD-1+ cells | Δ |
|---|---|---|---|
| SHZ400 | 0.71 | 8.14 | 11.59 |
| TVU155 | 0.85 | 2.51 | 2.96 |
| DMT806 | 4.57 | 12.78 | 2.79 |

*Example 9. In vivo anti-tumor efficacy of IL-21 PAC compounds in transgenic hPD-1/hIL-21R mice*

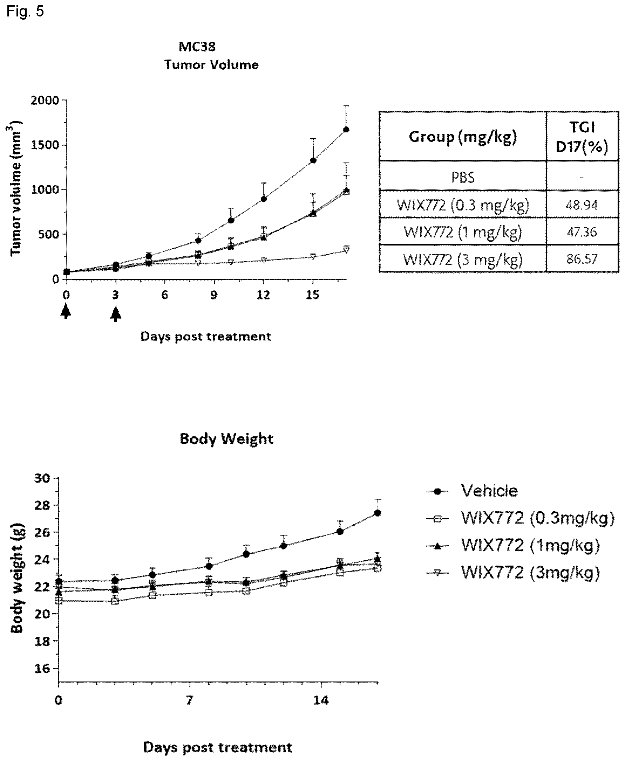

The MC38 colorectal cancer model was used to investigate the anti-tumor efficacy of selected IL-21 PAC compounds. Transgenic hPD-1/hIL-21R mice were injected s.c. in the right flank with 1x 10$^6$ MC38 cells. When tumors reached an average size of 70-100mm$^3$, compounds were administered i.v. (study day 0). A second compound administration was given i.v. on study day 3. Tumor volumes were measured daily with a caliper and calculated with the formula (length x width x width) 12. Tumor volume differences in % compared to vehicle at study end at day 17 after treatment onset were calculated. In a dose-intensification study WIX772 treatment was given at 0.3 mg/kg, 1mg/kg or 3mg/kg. Mice tolerated the compound at all administered doses with no body weight loss observed. Tumor growth inhibition (TGI) induced by WIX772 was dose-dependent with the highest effect at 3mg/kg, compared to treatment with vehicle (Figure 5, n=8 mice per group).

In a second study, multiple IL-21 PAC compounds were administered at 3 mg/kg on study day 0 and 3. TGI compared to treatment with vehicle was observed with all IL-21 PACs tested (Table 22).

Table 22 Tumor growth inhibition (TGI) by selected IL-21 PAC compounds compared to vehicle treatment on day 17 of IL-21PAC compounds on MC38 s.c. tumor bearing hPD-1/hIL-21R mice. Mice were treated on day 0 (tumor volume average 70-100mm$^3$) and day 3 i.v. with 3mg/kg of compound. n=7 mice per group.

| Treatment Group (mg/kg) | TGI D17(%) |
|---|---|
| Vehicle | - |
| EJD551 | 49.92 |
| KKY261 | 51.12 |
| LLX102 | 47.49 |
| SHZ400 | 62.27 |
| TVU155 | 34.78 |
| VUB931 | 54.61 |
| WBL338 | 75.01 |

*SEQUENCES:*

In the event of discrepancies between the sequences shown in the present specification and those of the enclosed sequence protocol according to WIPO standard ST.26, the sequences shown herein shall prevail.

CDR where indicated by underlining are assigned according to the Kabat numbering system

*SEQ ID NO 001: FYP014 heavy chain variable domain*

EVQLVESGGGLVKPGGSLRLSCAASGFIFSSYSMNWVRQAPGKGLEWVSSITSGSYYIHYADSV
KGRFTISRDNAKNSLYLQMNSLRAEDTAVYYCVRERGWGYYGMDVWGQGTTVTVSS

*SEQ ID NO 002: FYP014 light chain variable domain*

AIQLTQSPSSLSASVGDRVTITCRASQDIDSALAWYQQKPGKAPKILIHDASSLESGVPSRFSGSG
SGTDFTLTISSLQPEDFATYYCQQFNSYPYTFGQGTKLEIK

*SEQ ID NO 003: ZAP122 heavy chain variable domain*

QVQLQESGPGLVKPSETLSLTCTVS*GGSISSDF*WGWIRQPPGKGLEWIGY*ISSRGST*NYNPSLK
RRVTISVDTSRNQFSLKLSSVTAADTAVYYC*ARSAGVTDFDF*WGQGTLVTVSS

*SEQ ID NO 004: ZAP122 light chain variable domain*

DIQMTQSPSSVSASVGDRVTITCRAS*QGISSWL*AWYQHKPGKAPKLLIY*VA*SSLQSGVPSRFSG
SGSGTDFTLTISSLQPEDFATYYC*QQANSFPLT*FGGGTKVEIK

*SEQ ID NO 005: LKK631 heavy chain variable domain*

EVQLQQFGAEVVKPGASVKMSCKASGYTFTDYNMNWVKQSHEKSLEWIGDIHPKFHSTNYNQK
FKGKATLTIDTSSSTAYMELRNLTSEDTAVYYCARVGNYVYFVMDYWGQGTSVTVSS

*SEQ ID NO 006: LKK631 light chain variable domain*

SIVMTQTPKFLLVSAGDRVTITCKASQSVSNDVAWYQQKPGQSPKLLIYYASNRYTGVPDRFSG
SGYGTDFTFTISTVQAEDLAVYFCQQDYTSPWTFGGGTKLEIK

*SEQ ID NO 007: MIZ926 heavy chain variable domain*

EVQLVESGGGSVKPGGSLKLSCAASGLTFSHYYMYWVRQTPEKRLEWVAIISDGGDYTHYSDS
VKGRFTISRDNAKNNLYLQMSSLQSEDTAMYYCAGDYGYGYEGWFAYWGQGTLVTVST

*SEQ ID NO 008: MIZ926 light chain variable domain*

DIVMTQSQKFMSTSVGDRVSVTCKASQNVDTYVAWYQQKPGQSPKALIYSTSYRYSGVPDRFT
GSGSGTDFTLTISNVQSEDLAEYFCQQYNSYPWTFGGGTKLEIK

*SEQ ID NO 009 21A08Ap1 VH*

EVQLLESGGGLVQPGGSLRLSCAAS*GFTFSINA*MTWVRQAPGKGLEWVST*ISGSGFST*YYADSL
KGRFTISRDNSKNTLYLQMNSLRAEDTAVYYC*AKEVYGDY*WGQGTLVTVSS

*SEQ ID NO 010 21A08Ap1 VL*

RSVLTQPPSASGTPGQRVSISCSGA*SSNIGSQS*VFWYQQLPGTAPKLLIY*SNN*QRPSGVPDRFS
GSKSGTSASLAISGLRSEDEADYYC*AAWDDSLSIWV*FGGGTKLTVL

*SEQ ID NO 011 XVT458-z2-m6 VH*

QVQLVQSGAEVKKPGASVKVSCKAS*GYTFTNFY*IHWVRQAPGQGLEWMGS*IYPNYGIT*AYNQK
FKDRVTMTVDTSTSTAYMELRSLRSDDTAVYYC*ARGYSYAMDY*WGQGTTVTVSS

*SEQ ID NO 012 XVT458-z2-m6 VL*

DIQMTQSPSSVSASVGDRVTITCSASQGISGDLNWYQQKPGKAPKLLIYHTSQLHSDVPSRFSG
SGSGTDFTLTISSLQPEDFATYYCQGYSKDLLTFGGGTKLEIK

*SEQ ID NO 013 Anti-PD-1 antibody clone 21A08Ap1 heavy chain*
*CDRs; Italic: VH, regular: CH1-Hinge-CH2-CH3; **Fc silencing** bold: L232A, L233A, P327A; **Holes mutations (bold***

*italic) Y347C, T364S, L366A, Y405V*

*EVQLLESGGGLVQPGGSLRLSCAAS<u>GFTFSINAMT</u>WVRQAPGKGLEWVST<u>ISGSGFST</u>YYADSL*
*KGRFTISRDNSKNTLYLQMNSLRAEDTAVYYC<u>AKEVYGDY</u>WGQGTLVTVSS*ASTKGPSVFPLAP
SSKSTSGGTAALGCLVKDYFPEPVTVSWNSGALTSGVHTFPAVLQSSGLYSLSSVVTVPSSSLG
TQTYICNVNHKPSNTKVDKRVEPKSCDKTHTCPPCPAPE**AA**GGPSVFLFPPKPKDTLMISRTPE
VTCVVVDVSHEDPEVKFNWYVDGVEVHNAKTKPREEQYNSTYRVVSVLTVLHQDWLNGKEYK
CKVSNKAL**A**APIEKTISKAKGQPREPQV**C**TLPPSREEMTKNQVSL**SCA**VKGFYPSDIAVEWESN
GQPENNYKTTPPVLDSDGSFFL**V**SKLTVDKSRWQQGNVFSCSVMHEALHNHYTQKSLSLSPGK

*SEQ ID NO 014 Anti-PD-1 antibody clone 21A08Ap1 light chain*
*Italic VL*, regular *CL*

*RSVLTQPPSASGTPGQRVSISCSGASS<u>NIGSQS</u>VFWYQQLPGTAPKLLIY<u>SNN</u>QRPSGVPDRFS*
*GSKSGTSASLAISGLRSEDEADYYC<u>AAWDDSLSIWV</u>FGGGTKLTVL*GQPKAAPSVTLFPPSSEE
LQANKATLVCLISDFYPGAVTVAWKADSSPVKAGVETTTPSKQSNNKYAASSYLSLTPEQWKSH
RSYSCQVTHEGSTVEKTVAPTECS

*SEQ ID NO 015 mature hIL-21 sequence*

QGQDRHMIRMRQLIDIVDQLKNYVNDLVPEFLPAPEDVETNCEWSAFSCFQKAQLKSANTGNNE
RIINVSIKKLKRKPPSTNAGRRQKHRLTCPSCDSYEKKPPKEFLERFKSLLQKMIHQHLSSRTHGS
EDS

*SEQ ID NO 016 ZAP122 antibody HC*
<u>CDRs</u>; *Italic: VH,* regular: *CH1-Hinge-CH2-CH3;* **Fc silencing: L234A**, **L235A**, **P329A**; ***Knobs mutations S354C,***
***T366W***

*QVQLQESGPGLVKPSETLSLTCTVS<u>GGSISSDF</u>WGWIRQPPGKGLEWIGY<u>ISSRGST</u>NYNPSLK*
*RRVTISVDTSRNQFSLKLSSVTAADTAVYYC<u>ARSAGVTDFDF</u>WGQGTLVTVSS*ASTKGPSVFPL
APSSKSTSGGTAALGCLVKDYFPEPVTVSWNSGALTSGVHTFPAVLQSSGLYSLSSVVTVPSSS
LGTQTYICNVNHKPSNTKVDKRVEPKSCDKTHTCPPCPAPE**AA**GGPSVFLFPPKPKDTLMISRT
PEVTCVVVDVSHEDPEVKFNWYVDGVEVHNAKTKPREEQYNSTYRVVSVLTVLHQDWLNGKEY
KCKVSNKAL**A**APIEKTISKAKGQPREPQVYTLPP**C**REEMTKNQVSL**W**CLVKGFYPSDIAVEWES
NGQPENNYKTTPPVLDSDGSFFLYSKLTVDKSRWQQGNVFSCSVMHEALHNHYTQKSLSLSPG
K

*SEQ ID NO 017 ZAP122 antibody LC: VL (italics) - CL*

*DIQMTQSPSSVSASVGDRVTITCRAS<u>QGISSW</u>LAWYQHKPGKAPKLLIY<u>VA</u>SSLQSGVPSRFSG*
*SGSGTDFTLTISSLQPEDFATYYC<u>QQANSFPLT</u>FGGGTKVEIK*RTVAAPSVFIFPPSDEQLKSGTA
SVVCLLNNFYPREAKVQWKVDNALQSGNSQESVTEQDSKDSTYSLSSTLTLSKADYEKHKVYA
CEVTHQGLSSPVTKSFNRGEC

*SEQ ID NO 018XVT458-z2-m6 HC*
*Italic: VH,* regular: *CH1-Hinge-CH2-CH3*

*QVQLVQSGAEVKKPGASVKVSCKAS<u>GYTFTNFYIH</u>WVRQAPGQGLEWMGS<u>IYPNYGITA</u>YNQK<u>FKD</u>RVTMTVDTSTSTAYMELRSLRSDDTAVYYC<u>ARGYSYAMDY</u>WGQGTTVTVSS*ASTKGPSVF PLAPSSKSTSGGTAALGCLVKDYFPEPVTVSWNSGALTSGVHTFPAVLQSSGLYSLSSVVTVPS SSLGTQTYICNVNHKPSNTKVDKRVEPKSCDKTHTCPPCPAPEAAGGPSVFLFPPKPKDTLMISR TPEVTCVVVDVSHEDPEVKFNWYVDGVEVHNAKTKPREEQYNSTYRVVSVLTVLHQDWLNGKE YKCKVSNKALAAPIEKTISKAKGQPREPQVYTLPPSREEMTKNQVSLTCLVKGFYPSDIAVEWES NGQPENNYKTTPPVLDSDGSFFLYSKLTVDKSRWQQGNVFSCSVMHEALHNHYTQKSLSLSPG K

*SEQ ID NO 019 XVT458-z2-m6 LC VL (italics) - CL*

*DIQMTQSPSSVSASVGDRVTITCSAS<u>QGISGD</u>LNWYQQKPGKAPKLLIY<u>HTS</u>QLHSDVPSRFSG SGSGTDFTLTISSLQPEDFATYYC<u>QGYSKDLLT</u>FGGGTKLEIK*RTVAAPSVFIFPPSDEQLKSGTA SVVCLLNNFYPREAKVQWKVDNALQSGNSQESVTEQDSKDSTYSLSSTLTLSKADYEKHKVYA CEVTHQGLSSPVTKSFNRGEC

SEQ ID NO 020 Pembrolizumab LC variable domain

EIVLTQSPATLSLSPGERATLSCRASKGVSTSGYSYLHWYQQKPGQAPRLLIYLASYLESGVPAR FSGSGSGTDFTLTISSLEPEDFAVYYCQHSRDLPLTFGGGTKVEIK

SEQ ID NO 021 Pembrolizumab HC variable domain

QVQLVQSGVEVKKPGASVKVSCKASGYTFTNYYMYWVRQAPGQGLEWMGGINPSNGGTNFN EKFKNRVTLTTDSSTTTAYMELKSLQFDDTAVYYCARRDYRFDMGFDYWGQGTTVTVSS

SEQ ID NO 022 Nivolumab LC variable domain

EIVLTQSPATLSLSPGERATLSCRASQSVSSYLAWYQQKPGQAPRLLIYDASNRATGIPARFSGS GSGTDFTLTISSLEPEDFAVYYCQQSSNWPRTFGQGTKVEIK

SEQ ID NO 023 Nivolumab HC variable domain

QVQLVESGGGVVQPGRSLRLDCKASGITFSNSGMHWVRQAPGKGLEWVAVIWYDGSKRYYAD SVKGRFTISRDNSKNTLFLQMNSLRAEDTAVYYCATNDDYWGQGTLVTVSS

SEQ ID NO 024
SPNSASHSGSAPQTSSAPGSQ
SEQ ID NO 025
(G4S)4
SEQ ID NO 026
(G$_4$S)$_3$EAAAK
SEQ ID NO 027
(G4S)3
SEQ ID NO 028
G$_4$S
SEQ ID NO 029
GGSG
SEQ ID NO 030
(G$_4$S)$_2$

SEQ ID NO 031

GSG

SEQ ID NO 032

GGGSGG

SEQ ID NO 033

GGGSGGGS

SEQ ID NO 034

GS

SEQ ID NO 035 Prolgolimab VH

QVQLVQSGGGLVQPGGSLRLSCAASGFTFSSYWMYWVRQVPGKGLEWVSAIDTGGGRTYYA
DSVKGRFAISRVNAKNTMYLQMNSLRAEDTAVYYCARDEGGGTGWGVLKDWPYGLDAWGQG
TLVTVSS

SEQ ID NO 036 Prolgolimab VL

QPVLTQPLSVSVALGQTARITCGGNNIGSKNVHWYQQKPGQAPVLVIYRDSNRPSGIPERFSGS
NSGNTATLTISRAQAGDEADYYCQVWDSSTAVFGTGTKLTVL

SEQ ID NO 037 Zimberelimab VH

QLQLQESGPGLVKPSETLTLTCTVSADSISSTTYYWWIRQPPGKGLEWIGSISYSGSTYYNPSL
KSRVTVSVDTSKNQFSLKLNSVAATDTALYYCARHLGYNGRYLPFDYWGQGTLVTVSS

SEQ ID NO 038 Zimberelimab VL

QSALTQPASVSGSPGQSITISCTGTSSDVGFYNYVSWYQQHPGKAPELMIYDVSNRPSGVSDRF
SGSKSGNTASLTISGLQAEDEADYYCSSYTSISTWVFGGGTKLTVL

SEQ ID NO 039 AMG256 VH

EVQLVESGGSVVRPGGSLRLSCAASGFTVDDYSMSWVRQVPGKGLEWVSGINWNAGRTRYAD
AVKGRFTISRDSAKNSLYLQMNSLRAEDTALYYCAREFNNFESNWFDPWGQGTLVTVSS

SEQ ID NO 040 AMG256 VL

SYELTQPPSVSVSPGQTARITCSGDALPKKYAYWYQQKPGQAPVLVISEDAKRPSGIPERFSGS
SSGTMATLTISGAQVEDEADYYCYSTDASGNHRVFGGGTKLTVL

SEQ ID NO 041 human IgG1-Fc (CH1-Hinge-CH2-CH3)

ASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKRVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVMHEALHNHYT
QKSLSLSPGK

*Cited references:*

Shen S., et al. (2020) Front. Immunol. 8(11):832.
Davis et al., (2007) Nucleic Acids Res. 35(Web Server issue):W375-83.
Atwell, S. et al. (1997) J Mol Biol. 270(1):26-35.
Merchant, A. M., et al. (1998) Nat Biotechnol. 16(7):677-81.
Klein C, et al. (2019) Methods. 1;154:21-31.
Klein C, et al. (2016) MAbs 8(6):1010-20.
Bönisch M, et al. (2017) Protein Eng Des Sel. 30(9):685-696.
Bondensgaard K. (2007) J Biol Chem. 282(32):23326-36.

All scientific publications and patent documents cited in the present specification are incorporated by reference herein.

**Claims**

1. A proximity activated cytokine (PAC) comprising:

    - an anti-PD1 antibody antigen binding domain capable of binding to PD-1, comprising a heavy chain variable domain (PD1-VH), and a light chain variable domain (PD1-VL);
    - an anti-IL-21 antibody antigen binding domain, comprising:

        • a heavy chain variable domain (IL-21-VH); and
        • a light chain variable domain (IL-21-VL);
        and

    - an IL-21 polypeptide at least 98% identical to SEQ ID NO 015;

    wherein the IL-21 polypeptide is covalently linked to the IL-21-VL, or the IL-21-VH, to provide a single contiguous recombinant polypeptide.

2. The PAC according to claim 1, wherein the anti-IL-21 antibody antigen binding domain comprises:

    - an IL-21-VH comprising, or consisting of a polypeptide at least 95% identical to the sequence SEQ ID NO 003, particularly wherein said polypeptide at least 95% identical to the sequence SEQ ID NO 003 has at least one amino acid substitution disclosed in Table 3, or Table 4 compared to the sequence SEQ ID NO 003; and
    - an IL-21-VL comprising, or consisting of a polypeptide at least 95% identical to the sequence SEQ ID NO 004, particularly wherein said polypeptide at least 95% identical to the sequence SEQ ID NO 004 has at least one amino acid substitution disclosed in Table 3, or Table 4 compared to the sequence SEQ ID NO 004.

3. The PAC according to claim 1 or 2, wherein binding of pembrolizumab, or nivolumab to a PD-1+ cell does not significantly inhibit subsequent binding of the PAC to said cell.

4. The PAC according to any one of the claims 1 to 3, wherein

    - the PD1-VH comprises a variable domain comprising, or consisting of a polypeptide at least 95% identical to the sequence SEQ ID NO 009, particularly wherein the PD1-VH comprises a variable domain comprising, or consisting of SEQ ID NO 009; and
    - the PD1-VL comprises a variable domain comprising, or consisting of a polypeptide at least 95% identical to the sequence SEQ ID NO 010, particularly wherein the PD1-VL comprises a variable domain comprising, or consisting of SEQ ID NO 10, or a polypeptide having at least one amino acid substitution disclosed in Table 6 compared to SEQ ID NO 010.

5. The PAC according to any one of the claims 1 to 3, wherein

- the PD1-VH comprises a variable domain comprising, or consisting of a polypeptide at least 95% identical to the sequence SEQ ID NO 011, particularly wherein the PD1-VH comprises a variable domain comprising or consisting of SEQ ID NO 11, or a polypeptide having at least one amino acid substitution disclosed in Table 7 compared to the sequence SEQ ID NO 011; and

- the PD1-VL comprises a variable domain comprising, or consisting of a polypeptide at least 95% identical to the sequence SEQ ID NO 012, particularly wherein the PD1-VL comprises a variable domain comprising or consisting of SEQ ID NO 012, or a polypeptide having at least one amino acid substitution disclosed in Table 7 compared to the sequence SEQ ID NO 012.

6. The PAC accord to any one of the preceding claims, wherein the IL-21 polypeptide comprises or consists of SEQ ID NO 015.

7. The PAC accord to any one of the preceding claims, wherein an N-terminus of said IL-21-VL is covalently linked to a C-terminus of said IL-21 polypeptide by a peptide linker, particularly wherein the peptide linker has a length of 2 to 20 amino acids.

8. The PAC accord to any one of the claims 1 to 6, wherein an N-terminal residue and a C-terminal residue of said IL-21 polypeptide are embedded within a complementarity determining region (CDR) of the IL-21-VL, particularly wherein a polypeptide consisting of, listed from N-terminal to C-terminal direction, a peptide linker of 2 to 25 amino acids, an IL-21 polypeptide, and a peptide linker of 2 to 25 amino acids, is embedded within a CDR of the IL-21-VL.

9. The PAC accord to any one of the claims 1 to 6, wherein an N-terminus of said IL-21-VH is covalently linked to a C-terminus of said IL-21 polypeptide by a peptide linker, particularly wherein the peptide linker has a length of 10 to 25 amino acids.

10. An isolated nucleic acid encoding the PAC according to any one of the claims 1 to 9.

11. An expression vector comprising a nucleic acid encoding the PAC according to any one of claims 1 to 9 under control of a promoter operable in a mammalian cell.

12. The PAC according to any one of the claims 1 to 9, the isolated nucleic acid according to claim 10, or the expression vector according to claim 11, for use in treating cancer.

13. The PAC according to any one of the claims 3 to 9, for use in treating a cancer patient, wherein said cancer patient is receiving concurrent treatment with an anti-PD-1 antagonist antibody, particularly wherein said anti-PD-1 antagonist antibody is selected from the list consisting of nivolumab, pembrolizumab, dostarlimab, sintilimab, tislelizumab, cemiplimab, cetrelimab, or sasanlimab, more particularly wherein the anti-PD-1 antagonist antibody is selected from nivolumab or pembrolizumab.

14. An anti-PD-1 antagonist antibody for use in a cancer patient receiving concurrent treatment with the PAC according to any one of the claims 3 to 9.

15. A combination medicament comprising:

- the PAC as specified in any one of the claims 3 to 9, and
- an anti-PD-1 antagonist antibody, particularly wherein said anti-PD-1 antagonist antibody is selected from the list consisting of nivolumab, pembrolizumab, dostarlimab, sintilimab, tislelizumab, cemiplimab, cetrelimab, or sasanlimab, more particularly wherein the anti-PD-1 antagonist antibody is selected from nivolumab or pembrolizumab.

Fig. 1 A

Fig. 1 B

Figure 1 (continued)

C

Figure 1 (continued) D

Rechallange of cured animals

Age matched control

anti-mPD-1 targeted hIL-21 (0.3mg/kg)
+ PD-1 blocking mAb (10mg/kg)

Fig. 2

## Proximity-activated cytokine signalling

Fig. 3

Fig. 4

Fig. 5

**MC38**
**Tumor Volume**

| Group (mg/kg) | TGI D17(%) |
|---|---|
| PBS | - |
| WIX772 (0.3 mg/kg) | 48.94 |
| WIX772 (1 mg/kg) | 47.36 |
| WIX772 (3 mg/kg) | 86.57 |

**Body Weight**

- Vehicle
- WIX772 (0.3mg/kg)
- WIX772 (1mg/kg)
- WIX772 (3mg/kg)

Europäisches
Patentamt
European
Patent Office
Office européen
des brevets

# EUROPEAN SEARCH REPORT

**Application Number**

EP 24 20 8869

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/142471 A1 (ASKGENE PHARMA INC [US]) 15 July 2021 (2021-07-15) | 1-15 | INV. C07K16/24 |
| Y | * paragraph [0036]; figures 1D,1E,4; example 2; sequences 1-5,20,21 * | 2 | C07K16/28 C07K19/00 |
| X | WO 2023/052846 A2 (ILDONG PHARMACEUTICAL CO LTD [KR]) 6 April 2023 (2023-04-06) | 1-15 | |
| Y | * paragraph [0008]; figure 1 * | 2 | |
| Y | WO 2021/035188 A1 (ASKGENE PHARMA INC [US]) 25 February 2021 (2021-02-25) | 2 | |
| A | * paragraph [0036]; sequences 97-100 * | 1,3-15 | |
| X,P | WO 2024/133823 A1 (ANAVEON AG [CH]) 27 June 2024 (2024-06-27) * sequences 67,68,85,86 * | 1-15 | |
| A | WO 2019/028316 A1 (AMGEN INC [US]) 7 February 2019 (2019-02-07) * the whole document * | 1-15 | |

**TECHNICAL FIELDS SEARCHED (IPC)**

C07K
A61K

The present search report has been drawn up for all claims

| Place of search | Date of completion of the search | Examiner |
|---|---|---|
| Munich | 21 March 2025 | Cilensek, Zoran |

EPO FORM 1503 03.82 (P04C01)

# EP 4 671 273 A1

## ANNEX TO THE EUROPEAN SEARCH REPORT
## ON EUROPEAN PATENT APPLICATION NO.

EP 24 20 8869

21-03-2025

| Patent document cited in search report | Publication date | Patent family member(s) | Publication date |
|---|---|---|---|
| WO 2021142471 A1 | 15-07-2021 | AU 2021205981 A1 | 14-07-2022 |
| | | CA 3165927 A1 | 15-07-2021 |
| | | CN 114901679 A | 12-08-2022 |
| | | CN 119367553 A | 28-01-2025 |
| | | CN 119386199 A | 07-02-2025 |
| | | EP 4087864 A1 | 16-11-2022 |
| | | JP 2023509969 A | 10-03-2023 |
| | | US 2023108562 A1 | 06-04-2023 |
| | | WO 2021142471 A1 | 15-07-2021 |
| WO 2023052846 A2 | 06-04-2023 | AU 2022358565 A1 | 02-05-2024 |
| | | CA 3234007 A1 | 06-04-2023 |
| | | EP 4408877 A2 | 07-08-2024 |
| | | JP 2024537846 A | 16-10-2024 |
| | | KR 20240067113 A | 16-05-2024 |
| | | TW 202330581 A | 01-08-2023 |
| | | US 2023136331 A1 | 04-05-2023 |
| | | WO 2023052846 A2 | 06-04-2023 |
| WO 2021035188 A1 | 25-02-2021 | CN 115605504 A | 13-01-2023 |
| | | EP 4017594 A1 | 29-06-2022 |
| | | JP 2022545439 A | 27-10-2022 |
| | | US 2022289822 A1 | 15-09-2022 |
| | | WO 2021035188 A1 | 25-02-2021 |
| WO 2024133823 A1 | 27-06-2024 | WO 2024133823 A1 | 27-06-2024 |
| | | WO 2024133825 A1 | 27-06-2024 |
| WO 2019028316 A1 | 07-02-2019 | AU 2018311079 A1 | 13-02-2020 |
| | | AU 2022228122 A1 | 29-09-2022 |
| | | BR 112020002013 A2 | 28-07-2020 |
| | | BR 122021015266 B1 | 24-01-2023 |
| | | CA 3071376 A1 | 07-02-2019 |
| | | CL 2020000252 A1 | 21-08-2020 |
| | | CL 2022000300 A1 | 23-09-2022 |
| | | CL 2024002249 A1 | 21-02-2025 |
| | | CN 111164100 A | 15-05-2020 |
| | | CN 118126157 A | 04-06-2024 |
| | | CO 2020001113 A2 | 15-05-2020 |
| | | CR 20200099 A | 24-07-2020 |
| | | CY 1125429 T1 | 16-02-2024 |
| | | DK 3661954 T3 | 19-04-2022 |
| | | EP 3661954 A1 | 10-06-2020 |
| | | EP 4029877 A1 | 20-07-2022 |
| | | ES 2910969 T3 | 17-05-2022 |
| | | ES 2975221 T3 | 04-07-2024 |

EPO FORM P0459

page 1 of 2

## ANNEX TO THE EUROPEAN SEARCH REPORT
## ON EUROPEAN PATENT APPLICATION NO.

EP 24 20 8869

This annex lists the patent family members relating to the patent documents cited in the above-mentioned European search report.
The members are as contained in the European Patent Office EDP file on
The European Patent Office is in no way liable for these particulars which are merely given for the purpose of information.

21-03-2025

| Patent document cited in search report | Publication date | Patent family member(s) | Publication date |
|---|---|---|---|
| | | HR P20220404 T1 | 27-05-2022 |
| | | HU E058233 T2 | 28-07-2022 |
| | | IL 272137 A | 31-03-2020 |
| | | IL 312607 A | 01-07-2024 |
| | | JO P20200020 B1 | 17-09-2023 |
| | | JP 7079171 B2 | 01-06-2022 |
| | | JP 7531545 B2 | 09-08-2024 |
| | | JP 2019033743 A | 07-03-2019 |
| | | JP 2022116099 A | 09-08-2022 |
| | | KR 20200035291 A | 02-04-2020 |
| | | KR 20220092652 A | 01-07-2022 |
| | | LT 3661954 T | 11-04-2022 |
| | | MA 56289 B1 | 29-04-2022 |
| | | MY 195974 A | 27-02-2023 |
| | | PH 12020500231 A1 | 11-01-2021 |
| | | PL 3661954 T3 | 16-05-2022 |
| | | PT 3661954 T | 14-04-2022 |
| | | RS 63101 B1 | 29-04-2022 |
| | | SA 520411230 B1 | 08-11-2022 |
| | | SA 522432917 B1 | 05-11-2023 |
| | | SG 11202000821S A | 27-02-2020 |
| | | SI 3661954 T1 | 31-05-2022 |
| | | SM T202200162 T1 | 12-05-2022 |
| | | TN 2020000015 A1 | 04-10-2021 |
| | | TW 201920239 A | 01-06-2019 |
| | | TW 202246308 A | 01-12-2022 |
| | | TW 202342501 A | 01-11-2023 |
| | | US 2019046611 A1 | 14-02-2019 |
| | | US 2023381276 A1 | 30-11-2023 |
| | | UY 37829 A | 31-01-2019 |
| | | WO 2019028316 A1 | 07-02-2019 |

--------------------------------------------------------------------------

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 3103813 A1
- EP 3128997 B1

### Non-patent literature cited in the description

- **SAMBROOK et al.** Molecular Cloning: A Laboratory Manual. Cold Spring Harbor Laboratory Press, 2012
- **AUSUBEL et al.** Short Protocols in Molecular Biology. John Wiley & Sons, Inc, 2002
- **SMITH** ; **WATERMAN**. *Adv. Appl. Math*, 1981, vol. 2, 482
- **NEEDLEMAN** ; **WUNSCH**. *J. Mol. Biol.*, 1970, vol. 48, 443
- **PEARSON** ; **LIPMAN**. *Proc. Nat. Acad. Sci.*, 1988, vol. 85, 2444
- **ALTSCHUL et al.** *J. Mol. Biol.*, 1990, vol. 215, 403-410
- **STRYER**. Biochemistry, 21
- **HUSTON et al.** *PNAS*, 1988, vol. 85 (16), 5879-5883
- *CHEMICAL ABSTRACTS*, 946414-94-4
- *CHEMICAL ABSTRACTS*, 1374853-91-4
- *CHEMICAL ABSTRACTS*, 2022215-59-2
- *CHEMICAL ABSTRACTS*, 2072873-06-2
- *CHEMICAL ABSTRACTS*, 1858168-59-8
- *CHEMICAL ABSTRACTS*, 1801342-60-8
- *CHEMICAL ABSTRACTS*, 2050478-92-5
- *CHEMICAL ABSTRACTS*, 2206792-50-7
- *CHEMICAL ABSTRACTS*, 1924598-82-2
- *CHEMICAL ABSTRACTS*, 2315361-37-4
- *CHEMICAL ABSTRACTS*, 2249882-54-8
- **L. LACHMAN et al.** The Theory and Practice of Industrial Pharmacy. 2013
- **SHEN S et al.** *Front. Immunol*, 2020, vol. 8 (11), 832
- **DAVIS et al.** *Nucleic Acids Res.*, 2007, vol. 35, W375-83
- **ATWELL, S. et al.** *J Mol Biol.*, 1997, vol. 270 (1), 26-35
- **MERCHANT, A. M. et al.** *Nat Biotechnol.*, 1998, vol. 16 (7), 677-81
- **KLEIN C et al.** *Methods.*, 2019, vol. 1 (154), 21-31
- **KLEIN C et al.** *MAbs*, 2016, vol. 8 (6), 1010-20
- **BÖNISCH M et al.** *Protein Eng Des Sel.*, 2017, vol. 30 (9), 685-696
- **BONDENSGAARD K.** *J Biol Chem.*, 2007, vol. 282 (32), 23326-36